# EUROPEAN PATENT APPLICATION

(11) **EP 2 578 594 A1**
(43) Date of publication of application: **10.04.2013**
(21) Application number: 11306285.5
(22) Date of filing: 04.10.2011
(51) Int. Cl.: C07H 7/033, C07H 15/02, C07H 15/18, C07H 17/04

(54) **Process of preparation of L-iduronic acid comprising a decarboxylation/intramolecular cyclisation tandem reaction**

(71) Applicant: SANOFI, 75008 Paris (FR)
(72) Inventor: Salamone, Stephane, 75015 Paris (FR); Chapleur, Yves, 54506 Vandoeuvre les Nancy (FR); Boisbrun, Michel, 54506 Vandoeuvre Les Nancy (FR)
(74) Representative: Jacobson, Claude

(57) **Abstract**

The present invention relates to a process of preparation of L-iduronic acid and derivatives comprising a decarboxylation/intramolecular cyclisation tandem reaction. The present invention also relates to the intermediates of the process, as well as their use as intermediates in the preparation of Idraparinux.

## Description

In the early 1980's, scientifics have discovered that the antithrombotic activity of heparin is due to a pentasaccharide fragment constituting heparin, as referred to as **DEFGH,** where the **G** unit is an L-iduronic acid derivative. As L-iduronic acid is a very rare natural molecule, its derivatives should be synthesized from abundant and inexpensive substrates, in a minimum of steps. Thus, the preparation of L-iduronic acid units is one of the major stakes in the synthesis of heparin derivatives.

Several synthetic pentasaccharides have been developed, such as ldraparinux, where all hydroxyl groups are methylated or sulphated, as illustrated below:

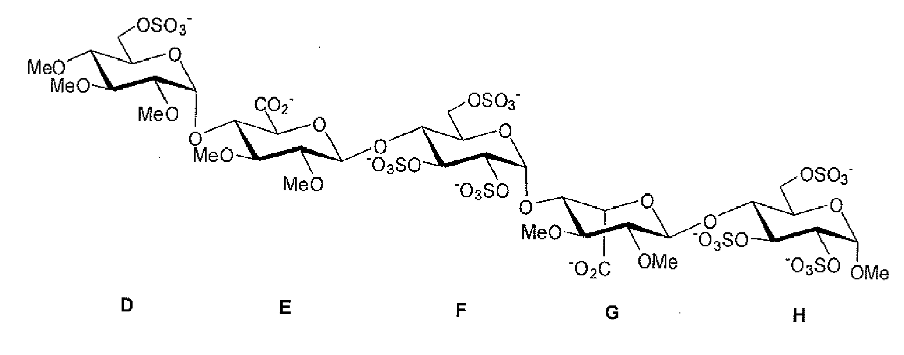

Initially, the firm Organon developed a way of synthesis for the preparation of the "active pentasaccharide". This synthesis, using the 3-*O*-benzyl-1,2-*O*-isopropylidene-α-D-glucofuranose as substrate (Van Boeckel et al., J. Carbohydr. Chem. 1985, 4, p.293-321), comprises more than 50 steps, and the inversion of configuration of the C5 carbon is carried out by the opening of an epoxide. After a step of protection followed by a bromination, the **G** unit is thus obtained. It is well known that the synthesis of said **G** unit is very tedious, due to the number of steps for obtaining such unit and the known tendency of L-idose derivatives to exist as furanoses. After being coupled to the **H** unit, successive steps of protection-deprotection then an oxidation reaction carried out on C6 carbon, lead to the **GH** disaccharide.

In the preparation of Idraparinux, the synthesis of the disaccharide **GH** is nearly similar to the above synthesis of early synthetic pentasaccharides. The major innovation lies in the obtaining of disaccharide **EF** by epimerization of disaccharide **GH**. The coupling of both disaccharides leads to the tetrasaccharide **EFGH**, which is further coupled to the **D** unit for obtaining said pentasaccharide. The preparation of the disaccharide **EF** from **GH** allows notably the decrease of the total number of the steps to approximatively 25 (Petitou, M.; Van Boeckel, C.A. Angew. Chem., Int.Ed. 2004, 43, p.3118-3133).

Hence, all current syntheses of the "active pentasaccharide" comprise a large number of steps and more particularly involves the complex synthesis of key L-iduronic acid derivative (**G** unit). Indeed, the preparation of the **G** unit of the "active pentasaccharide" of heparin has always been a limiting step in the synthesis of antithrombotic heparin derivatives.

Thus, there is still a need for a new efficient process of preparation of L-iduronic acid derivative, which would not possess the drawbacks established above and would be compatible with industrial scales. Besides, there is a need for such process which would in addition lead to an improved process of preparation of the "active pentasaccharide" constituting the heparin derivatives.

The inventors have now found a process of preparation of a L-iduronic acid unit from a D-glucuronic acid derivative by directly inverting the configuration of the carbon C5. The process according to the invention advantageously avoids the tedious steps as described in the prior art and is thus easier to carry out. The process according to the invention allows a significant decrease of the number of steps and improvement of the overall yield.

The process for preparing L-iduronic acid derivative according to the invention involves the intermediate of formula (VII) : 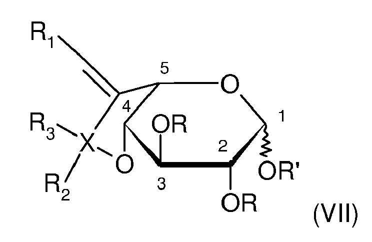 wherein :
- R, each being independently of each other identical or different, chosen from the group consisting in : alkyl, aryl and -arylalkyl;
- R' is chosen from the group consisting in : alkyl, aryl, -arylalkyl and oligosaccharide;
- R₁ represents H or alkyl;
- R₂ represents H or -O-alkyl;
- R₃ represents H or -O-alkyl;
- X represents C or Si; provided that when X represents Si, then R₃ is alkyl or when X represents C, then C is an asymmetric carbon which may possess either the (R) or the (S) configuration;
- 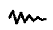 represents either α or β.

According to a first object, the present invention concerns a process for preparing a compound of formula (VII) : 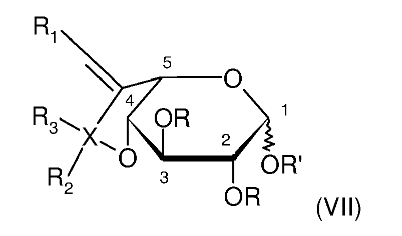 comprising conducting a decarboxylation/intramolecular cyclisation tandem reaction, which allows the inversion of configuration of carbon C5 from a compound of formula (VI) 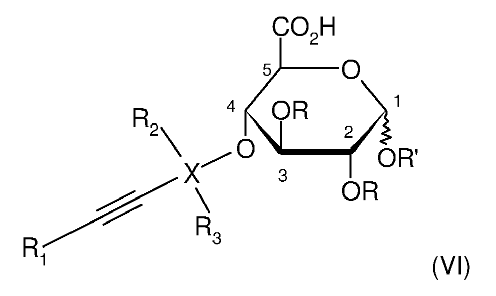 wherein :
- R, each being independently of each other identical or different, chosen from the group consisting in : alkyl, aryl and -arylalkyl;
- R' is chosen from the group consisting in : alkyl, aryl, -arylalkyl and oligosaccharide;
- R₁ represents H or alkyl;
- R₂ represents H or -O-alkyl;
- R₃ represents H or -O-alkyl;
- X represents C or Si; provided that when X represents Si, then R₃ is alkyl or when X represents C, then C is an asymmetric carbon which may possess either the (R) or the (S) configuration;
- 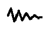 represents either α or β.

As used herein, unless otherwise specified, the expression "oligosaccharide" relates to a chain, with any degree of branching, of from one to ten monosaccharide units linked via glycosidic bonds.

As used herein, unless otherwise specified, the expression "tandem reaction" designates a consecutive series of intramolecular reactions which are carried out without isolating intermediates.

As used herein, unless otherwise specified, the expression "decarboxylation/intramolecular cyclisation tandem reaction" designates a tandem reaction of a decarboxylation reaction followed by an intramolecular cyclisation reaction, without isolating the intermediate obtained after the decarboxylation reaction.

As used herein, unless otherwise specified, the term "alkyl" means an aliphatic hydrocarbon group which may be straight or branched chain having about 1 to about 10 carbon atoms in the chain. Particular alkyl groups have from 1 to about 6 carbon atoms. Examples of alkyl include but are not limited to methyl, ethyl, n-propyl, i-propyl, n-butyl, s-butyl, t-butyl, n-pentyl, heptyl, octyl, nonyl and decyl.

As used herein, unless otherwise specified, the term "aryl" refers to monocyclic or bicyclic aromatic groups containing from 6 to 12 carbons in the ring portion, In particular, 6 to 10 carbons in the ring portion. Exemplary aryl groups include phenyl.

As used herein, unless otherwise specified, the term "arylalkyl" means an arylalkyl-group in which the aryl and alkyl moieties are as previously described. Exemplary arylalkyl groups include benzyl.

The compounds referred to herein may contain one or more assymetric carbon atoms, and may be isolated in optically or racemic forms. They can exist as enantiomer or diastereomer. Thus, enantiomer, diastereomer, and their mixture are part of the present invention.

The twisted bond 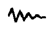 borne by the carbon C1 represents a bond either above or below the plane of the six-membered ring to which it is attached. The twisted bond on carbon C1 thus represents either the α or the β anomeric conformation of said carbon.

According to a particular embodiment, oligosaccharides include monosaccharides, and the following monosaccharides may be cited : 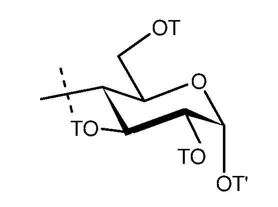 wherein T and T' being independently of each other identical or different, chosen from the group consisting in : alkyl, aryl and ―arylalkyl, more particularly T and T' are protecting groups of the hydroxyl functions. In particular, T and T' may be chosen from alkyl or ―arylalkyl, more particularly T is benzyl and T' is methyl.

In a particular embodiment, the oligosaccharide wherein T represents benzyl and T' represents methyl, may be cited.

According to a particular embodiment, in formulae (VI) and (VII) :
- R₁ represents H; and/or
- R₂ represents -O-ethyl; and/or
- R₃ represents H; and/or
- X is C; and/or
- R' represents methyl or an oligosaccharide as defined above; and/or
- R represents methyl.

The invention also concerns a process for preparing a compound of formula (IX) from a compound of formula (IV), which comprises the process for preparing the compound of formula (VII) from the compound of formula (VI) as defined above.

According to another object, the present invention thus concerns a process for preparing a compound of formula (IX) : 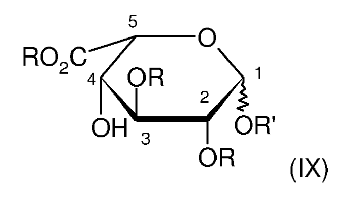 comprising reacting a compound of formula (IV): 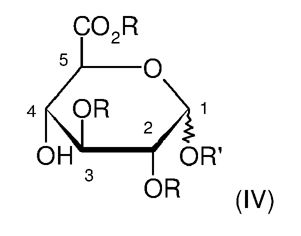 in which, the twisted bond 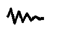 , R and R' are as defined above, wherein said process comprises the process of preparation of a compound of formula (VII) from a compound of formula (VI) by the decarboxylation/intramolecular cyclisation tandem reaction as defined above.

According to the present invention, the decarboxylation reaction is a radical decarboxylation chosen from the Barton decarboxylation, Kolbe decarboxylation or the decarboxylation via a *N*-(acyloxy)phthalimide.

The decarboxylation via a *N*-(acyloxy)phthalimide.may be carried out by application or adaptation of the method described by Okada et al. (J. Am. Chem. Soc. 1988, 110, p.8736-8738).

The Kolbe decarboxylation may be carried out by application or adaptation of the method described by Schäffer et al. (Pure Appl. Chem. 2007, 79, p.2047-2057).

In a particular embodiment, the decarboxylation is a Barton decarboxylation.

Typically, the Barton decarboxylation proceeds via the activation of a carboxylic acid in the form of a thiohydroxamate ester, also known as Barton ester. The activation may be carried out in presence of a compound such as 2-mercaptopyridine N-oxide sodium salt or *N*-hydroxypyridine-2-thione. Then, the homolytic N-O bond cleavage may occur upon heating, by irradiation with light or in presence of a radical initiator, in order to initiate the reaction.

In particular, the Barton ester may be obtained *via* activation of the carboxylic acid with 2-mercaptopyridine N-oxide sodium salt. Generally, in this reaction, the carboxylic acid may be activated by action of an activating agent chosen from the group of acyl chloride, mixed anhydride acyloxyphosphonium, acyloxyuronium, activated esters such as succinimide ester, p-nitrophenyl ester, or with the aid of coupling agents such as carbodiimides. Typically, the carboxylic acid of compound (VI) may be activated by action of an activating agent, more particularly an acyl chloride, such as methyl chloroformate, acetyl chloride, oxalyl chloride, ethyl chloroformate, isobutylchloroformate, butyryl chloride, isobutyryl chloride. Particularly, the carboxylic acid of compound (VI) is activated by the isobutylchloroformate (IBCF).

In the case of reductive decarboxylation, a hydrogen donor may be used in the reaction as radical trapping species, such as Bu₃SnH, t-BuSH, PhSH, Ph₂SiH₂ orTMS₃SiH. In particular, the decarboxylation may be carried out with *t*-BuSH.

Typically, the radical trapping species may be introduced in quantities such as the number of equivalent of the radical trapping species is comprised between 1.1 and 2 equivalents. In particular, the decarboxylation reaction is carried out with 1.2 equivalent of t-BuSH. NaOCl may be used at the end of the reaction for neutralizing *tert-*butylthiol.

Generally, the decarboxylation reaction may be carried out in presence of an organic base chosen from: triethylamine, diisopropylethylamine, N-methylmorpholine, piperidine, 1,8-diazabicyclo[5.4.0]undec-7-ene. In particular, N-methylmorpholine is used.

According to the invention, the decarboxylation/intramolecular cyclisation tandem reaction may be carried out under UV irradiations.

In general, the activating agent is introduced in the reaction medium at a temperature comprised between -10°C and 25°C. In particular, the reaction is performed at 0°C.

Typically, the solvent used for the decarboxylation reaction is a polar solvent such as THF or acetonitrile, or in a non polar solvent as chloroform, benzene, chlorobenzene or toluene, dichloromethane, carbon tetrachloride, xylene. The use of a radical trapping species, such as tert-butylthiol, may be avoided when chloroform is used as solvent, according to Ko et al. (Org. Lett. 2011, 13(8), p.1944-1947).

According to the invention, the decarboxylation reaction is performed in THF.

The decarboxylation may be carried out by application or adaptation of the method described by Barton et al. (Tetrahedron: Asymmetry 1994, vol. 5, n°11, p.2123-2136). However, Barton *et al.* disclose a decarboxylation/intermolecular cyclisation tandem reaction which leads to a mixture of L-idose and D-glucose, which are epimers.

In the present invention, the inventor advantageously found a new method that allows the formation of the L-idose epimer and not a mixture of both epimers. The method is based on a decarboxylation/intramolecular cyclisation tandem reaction. Such intramolecular cyclisation has never been described in said tandem reaction.

According to another object, the process of preparation of the compound of formula (IX) from the compound of formula (IV) may comprise the preparation of a compound of formula (VI) from a compound of formula (IV).

Therefore, the process according to the invention may comprise preparing the compound of formula (VI) above, comprising:
a. the transacetalisation of the compound of formula (IV) above for preparing a compound of formula (V) : 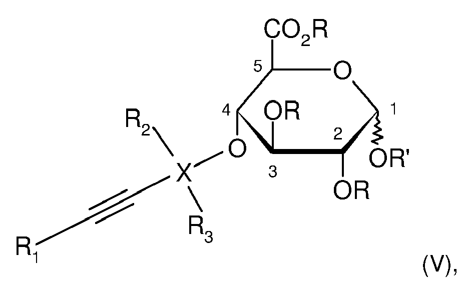 and
b. the saponification of the compound of formula (V) resulting from the step a) for preparing the compound of formula (VI): 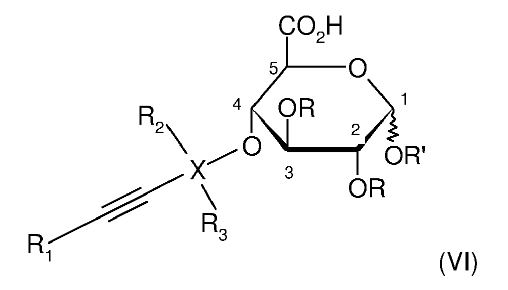
wherein 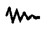 , R, R', X, R₁, R₂ and R₃ are as defined above.

The transacetalisation step may be carried out by application or adaptation of a known method such as described by Rodriguez-Fernandez et al. (Synlett 2007, n°11, p. 1703-1706).

In general, the transacetalisation may be carried out in an aprotic solvent such as chloroform, dichloromethane, toluene, acetonitrile, THF, DMF, DMSO, benzene, dioxane. In particular, the solvent is chloroform.

Typically, the transacetalisation is driven to the acetal when ethanol is removed from the reaction medium either by azeotropic distillation or trapping with molecular sieves or dessicant. According to the invention, the transacetalisation is generally carried out in presence of a dessicant, and acidic catalyst such as P₂O₅.

Typically, the reaction medium is carried out at a temperature range between 25° and 90°C. Particularly, the temperature of the transacetalisation is 60°C.

In general, the reaction is stirred for several hours. Particularly, the reaction is stirred approximately for four to five hours.

Typically, the saponification reaction may be carried out in presence of a strong base, such as NaOH, KOH, LiOH, CsOH, Ba(OH)₂, Mg(OH)₂, Ca(OH)₂. In particular, NaOH is used.

The strong base may be used in excess compared to compound (V). In particular, between 1 and 3 equivalents of NaOH are used in the saponification reaction. More particularly, 1.3 equivalents of NaOH are used.

In general, the saponification reaction may be performed for several hours at a temperature range between 0°C and 60°C. In particular, the reaction is carried out at room temperature, such as between 20°C and 30°C, for approximately five hours.

According to another object, the process of preparation of the compound of formula (IX) from the compound of formula (IV) may comprise the preparation of the compound of formula (IX) from a compound of formula (VI).

Therefore, the process according to the invention may further comprise preparing the compound of formula (IX) above, comprising:
a. the ozonolysis of a compound of formula (VII) : 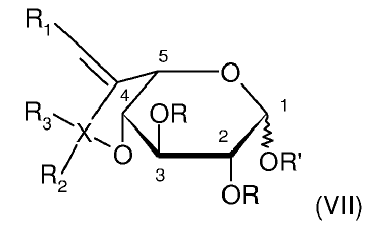 for preparing a compound of formula (VIII): 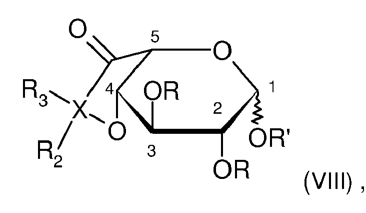 and
b. the oxidation/alkylation sequential reactions of the compound of formula (VIII) resulting from step a), for preparing the compound of formula (IX) : 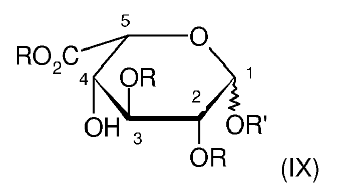 wherein 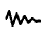 , R, R', X, R₁, R₂ and R₃ are as defined above.

In a typical procedure, ozone is bubbled through a solution of the compound of formula (VII) comprising an alkene moiety, in a solvent, chosen among methanol, ethanol or dichloromethane, at ―78 °C until the solution takes on a characteristic blue color. This indicates consumption of the alkene. Generally, a reagent chosen among triphenylphosphine, thiourea, zinc or dimethylsulfide, is then added to convert the intermediate ozonide to a carbonyl derivative.

Typically, the ozonolysis step may be carried out by application or adaptation of a known method such as described by Frezza et al. (Eur. J. Org. Chem. 2006, 4731-4736), namely by using ozone and the dimethylsulfide as reagent.

According to the present invention, the Baeyer-Villiger oxidation of the ketone function in C6 is carried out.

Generally, the Baeyer-Villiger oxidation may be carried out either in presence of peracid chosen from m-CPBA, peroxyacetic acid or peroxytrifluoroacetic acid, or either with hydrogen peroxide with a Lewis acid.

The oxidation step may be carried out by application or adaptation of a known method such as described by Finet et al. (Eur. J. Org. Chem. 2007, 4116-4123), namely by using m-CPBA as peracid, and in presence of the base NaHCO₃

According to the process of the present invention, the esterification step may be carried out directly after the oxidation step without any purification. Indeed, the intermediate lactone is cleaved *in situ* to give a mixture of 4-hydroxyl, 6-carboxylic acid compound and a 4-carbonate, 6-carboxylic acid compound. This mixture is treated in soft acidic conditions to cleave the carbonate and give a unique carboxylic acid which is esterified. Thus, the alkylation step may be performed on the crude residue resulting from the previous oxidation step.

As used herein, unless otherwise specified, the expression "sequential reaction" designates a series of consecutive reactions which are carried out without isolating the formed intermediates.

As used herein, unless otherwise specified, the expression "oxidation/alkylation sequential reaction" means the sequential reaction of an oxidation reaction followed by an alkylation reaction, without isolating the formed intermediates.

Typically, an esterification of a carboxylic acid moiety may be carried out by any known method, and in particular in presence of an alcohol such as methanol, ethanol, propanol, isopropanol, butanol, isobutanol, tertbutanol, with an acid catalyst such as *para-*toluenesulfonic acid. The esterification reaction may also be carried out in presence of an alcohol, dicyclohexylcarbodiimide (DCC) and the catalyst 4-dimethylaminopyridine (DMAP).

Typically, the esterification reaction may also be carried out in presence of an alkylating agent selected from the group of alkyl halides or alkyl triflates, such as methyl iodide, ethyl iodide, propyl iodide, butyl iodide, methyl trifalte, ethyl triflate, butyl triflate, methyl chloride, ethyl chloride, butyl chloride, methyl bromide, ethyl bromide, propyl bromide, butyl bromide, and a base chosen from triethylamine, diisopropylethylamine, N-methylmorpholine, piperidine. In the present invention, triethylamine may be used as base and Mel as alkylating agent.

Classically, the solvent which may be used for the esterification step is chosen among THF, dichloromethane, acetonitrile, toluene, dioxane, DMF, dichloroethane, chloroform, diethylether. In particular, DMF is used.

Typically, the alkylating agent and the base may be used in excess compared to compound of formula (VIII). The quantity of alkylating agent may be comprised between 2 and 8 equivalents compared to compound of formula (VIII). In particular, 5 equivalents of Mel are used. In general, the quantity of the base may be comprised between 1 and 5 equivalents compared to compound of formula (III). In particular, 1.1 equivalents of triethylamine are used.

Generally, the reaction may be stirred for several hours at a temperature range comprised between 0°C and 25°C. In particular, the reaction medium is stirred for approximately 3.5 hours at about 25°C.

According to another object, the process of preparation of the compound of formula (IX) from the compound of formula (IV) may comprise a preliminary step for preparing the compound of formula (IV) from a compound of formula (III).

Therefore, the process of the invention may further comprise the preliminary preparation of the compound of formula (IV) : 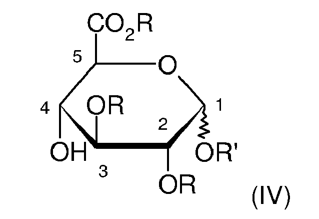 by oxidation/esterification sequential reactions of a compound of formula (III) : 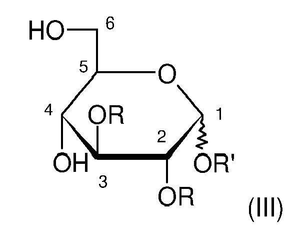 wherein 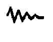 , R and R' are as defined above.

According to the invention, the intermediate resulting from the oxidation step of the hydroxyl function borne by the carbon C6 of compound (III) may not be isolated. The intermediate may be directly involved in the following step consisting in the esterification of the carboxylic acid moiety leading to compound of formula (IV).

This reaction may be carried out by application or adaptation of a known method such as described by de Nooy et al. (Tetrahedron 1995, vol. 51, n°29, p.8023-8032). Nooy *et al.* describe an oxidation of primary alcohols in presence of the system TEMPO-NaBr-NaOCl.

Typically, the oxidation may be carried out in the presence of a catalyst, such as (2,2,6,6-Tetramethylpiperidin-1-yl)oxyl (TEMPO), 4-hydroxy-TEMPO, 4-acetamido-TEMPO. In the present invention, TEMPO is used as catalyst. Thus, usually, the catalyst may be introduced in the reaction medium in quantity comprised between 0.01 and 0.6 equivalents. In particular, the TEMPO catalyst is used in 0.02 equivalents compared to compound (III).

According to the invention, the oxidation step may be carried out in presence of an oxidant, such as NaOCl, and in presence of a catalyst, such as NaBr.

Typically, the oxidant may be used in stoichiometry or in excess compared to the compound to be oxidized. In particular, 4 equivalents of NaOCl are used.

In general, the oxidation may be carried out in aqueous medium, in a biphasic medium or in an organic solvent chosen from dichloromethane, chloroform, toluene, acetonitrile. In particular, the oxidation is set up in water.

Typically, the oxidation of a primary alcohol may be carried out in a temperature range comprised between -10°C and 25°C. In particular, the oxidation step may be carried out at approximately 0°C.

Usually, the reaction medium may be acidified at the end of the reaction for obtaining the desired product. In particular, hydrochloric acid is used to acidify the reaction medium.

According to the invention, the esterification of the product resulting from the oxidation of a compound of formula (III) may be carried out without isolation of said product.

Typically, an esterification of a carboxylic acid moiety may be carried out in presence of an alcohol such as methanol, ethanol, propanol, isopropanol, butanol, isobutanol, tertbutanol ...

Advantageously, the solvent of the reaction may also be used as a reagent. In particular, methanol is used as reagent.

Typically, an acid catalyst may be used in the esterification reaction. Mention may be made of sulfuric acid, hydrochloric acid, or para-toluenesulfonic acid, camphorsulfonic acid. In particular, para-toluenesulfonic acid may be used in the present invention. In general, between 0.1 and 0.6 equivalents of catalyst may be used compared to the compound resulting from the oxidation step. In particular, 0.2 equivalents of *para-*toluenesulfonic acid are used.

Usually, the temperature of the esterification step may be comprised between 25°C and 80°C. In particular, the reaction is carried out under reflux of the methanol (65°C).

According to another object, the process of preparation of the compound of formula (IX) from the compound of formula (IV) may comprise a preliminary step for preparing the compound of formula (III) from a compound of formula (II).

Therefore, the process of the invention may further comprise the preliminary preparation of the compound of formula (III) : 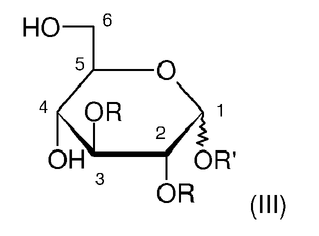 by deprotection of the 4,6-benzylidene protection of a compound of formula (II) : 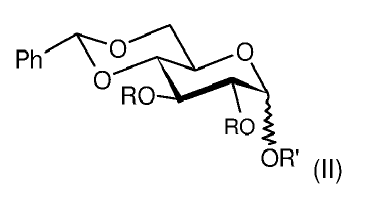 wherein 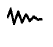 , R are as defined above and R' is chosen from the group consisting in: alkyl, aryl, -arylalkyl.

Generally, the 4,6-*O*-benzylidene acetal protective group may be removed by mild acid hydrolysis or by catalytic hydrogenolysis.

Typically, the acid catalyst may be chosen among camphorsulfonic acid, *para-*toluenesulfonic acid, para-toluenesulfonic acid monohydrate, HCl, HNO₃ H₂SO₄, H₃PO₄-In particular, the para-toluenesulfonic acid monohydrate is used.

Typically, the deprotection of a benzylidene acetal may be carried out in a protic solvent such as methanol, ethanol, water, isopropanol, *tert-*butanol. In particular, the solvent is methanol.

Generally, the quantity of the acid catalyst is comprised between 0.01 and 0.8 equivalents compared to the compound of formula (II). In particular, 0.05 equivalent of catalyst is used.

According to another object, the process of preparation of the compound of formula (IX) from the compound of formula (IV) may comprise a preliminary step for preparing the compound of formula (II) from a compound of formula (I).

Therefore, the process of the invention may further comprise the preliminary preparation of the compound of formula (II) : 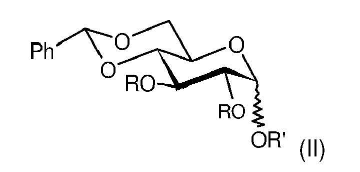 by O-alkylation of a compound of formula (I): 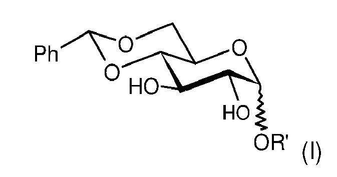 wherein 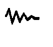 , R are as defined above and R' is chosen from the group consisting in: alkyl, aryl, -arylalkyl.

Typically, the *O*-alkylation reaction may be carried out in presence of an alkylating agent selected from the group of alkyl halides or alkyl triflates, such as methyl iodide, ethyl iodide, propyl iodide, butyl iodide, methyl trifalte, ethyl triflate, butyl triflate, methyl chloride, ethyl chloride, propyl chloride, butyl chloride, methyl bromide, ethyl bromide, propyl bromide, butyl bromide, and a base chosen from sodium hydride, lithium hydride, potassium hydride, KOH and NaOH. In the present invention, NaH may be used as base and Mel as alkylating agent.

Generally, the solvent which may be used for the alkylation step is chosen among THF, acetonitrile, diethyl ether, dioxane, DMF, DMSO. In particular, THF is used.

Typically, the alkylating agent and the base may be used in excess compared to compound of formula (I) and it may depend on the number of hydroxyl groups which may be alkylated. In the present invention, a compound of formula (I) possesses two hydroxyl groups that may be alkylated. The quantity of alkylating agent may be comprised between 2 and 5 equivalents compared to compound of formula (III). In particular, 2.8 equivalents of Mel are used. In general, the quantity of the base may be comprised between 2 and 5 equivalents compared to compound of formula (III). In particular, 2.5 equivalents of NaH are used.

Generally, the reaction may be stirred for several hours at a temperature range comprised between 0°C and 25°C. In particular, the reaction medium is stirred for approximately 16 hours at 25°C.

According to an other aspect, the process may further comprise preparing a compound of formula (I) : 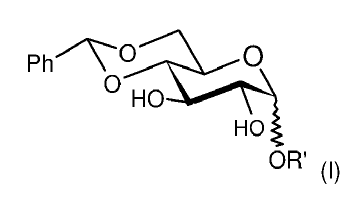 comprising a 4,6-*O*-benzylidene protection of a compound of formula (A) 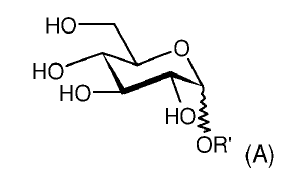 wherein 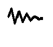 , R is as defined above and R' is chosen from the group consisting in: alkyl, aryl, -arylalkyl.

Typically, the use of benzylidene acetals as protecting groups in carbohydrate chemistry is utterly important. Generally, the 4,6-O-benzylidene protection of the hydroxyl groups in C4 and C6 may be carried out in presence of benzaldehyde and zinc chloride, or in presence of the system consisting in benzaldehyde dimethyl acetal with catalytic amount of acid.

In the present invention, the use of benzaldehyde and zinc chloride is preferred.

Advantageously, benzaldehyde is used both as reagent and as solvent. Typically, the quantity of benzaldehyde may be comprised between 2 and 10 equivalents. In particular, 5.9 equivalents of benzaldehyde are used compared to the compound of formula (A).

Generally, the reaction may be stirred for several hours at a temperature range comprised between 10°C and 35°C. In particular, the reaction medium is stirred for approximately 16 hours at 25°C.

The compound of formula (A), wherein R' is chosen from the group consisting in: alkyl, aryl and ―arylalkyl, and in particular the methyl-α-D-glucopyranoside, is commercialized by chemical company, such as Aldrich.

According to a particular embodiment, in the compounds (IV), (V), (VI), (VII), (VIII) and (IX), referred herein:
- R₁ represents H; and/or
- R₂ represents -O-ethyl; and/or
- R₃ represents H; and/or
- X is C; and/or
- R' represents methyl or an oligosaccharide as defined above; and/or
- R represents methyl.

According to a particular embodiment, in formulae (V), (VI), (VII), (VIII) and (IX) above:
when R' is chosen from the group consisting in : alkyl, aryl and -arylalkyl, then the twisted bond 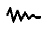 is in α position, as in : 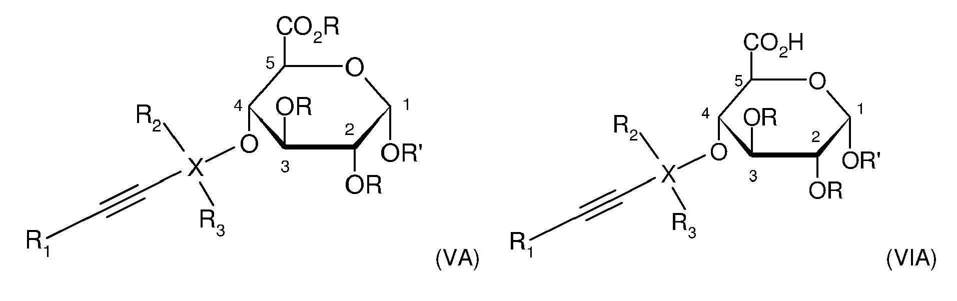 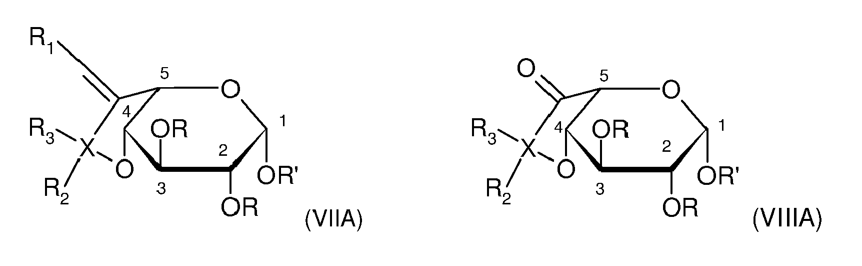 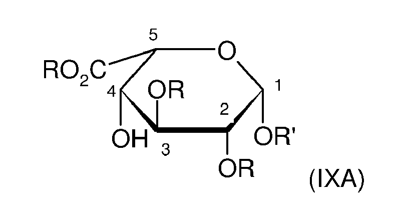 or when R' is the monosaccharide as depicted above, then the twisted bond 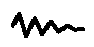 is in β position, as in : 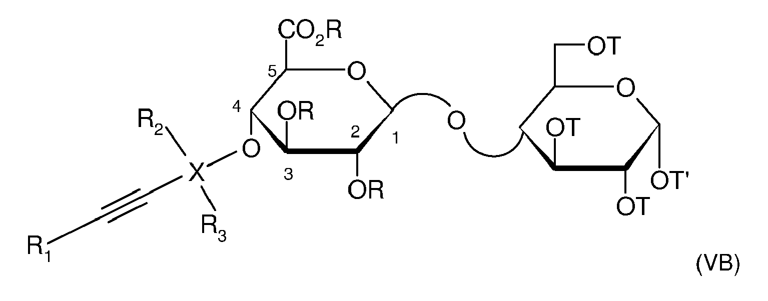 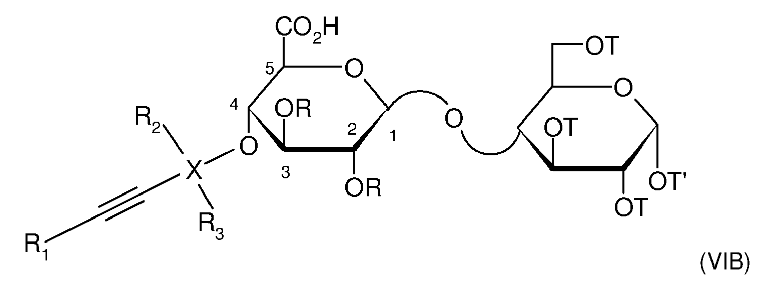 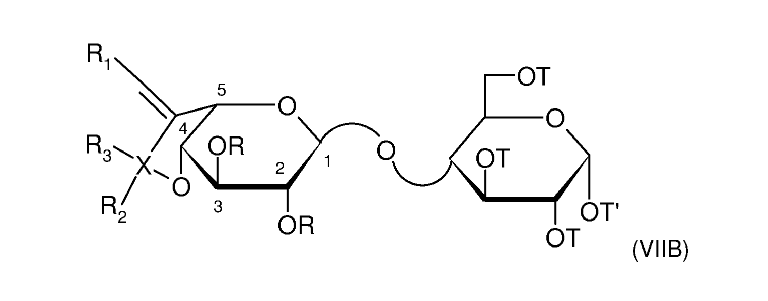 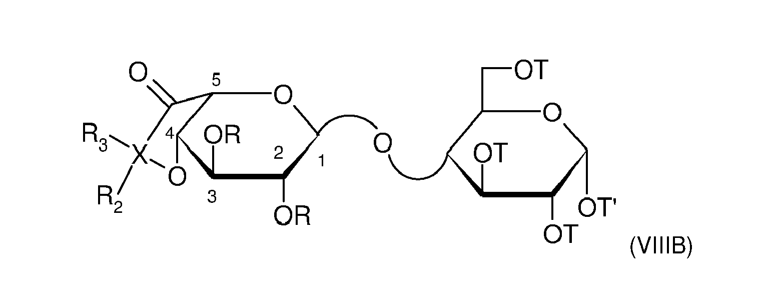 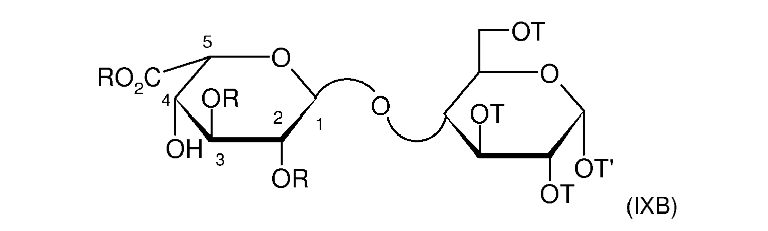
wherein R, X, R₁, R₂, R₃, T and T' are as defined above.

The process as defined above may be illustrated by the following scheme :

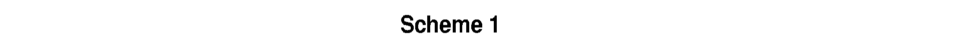

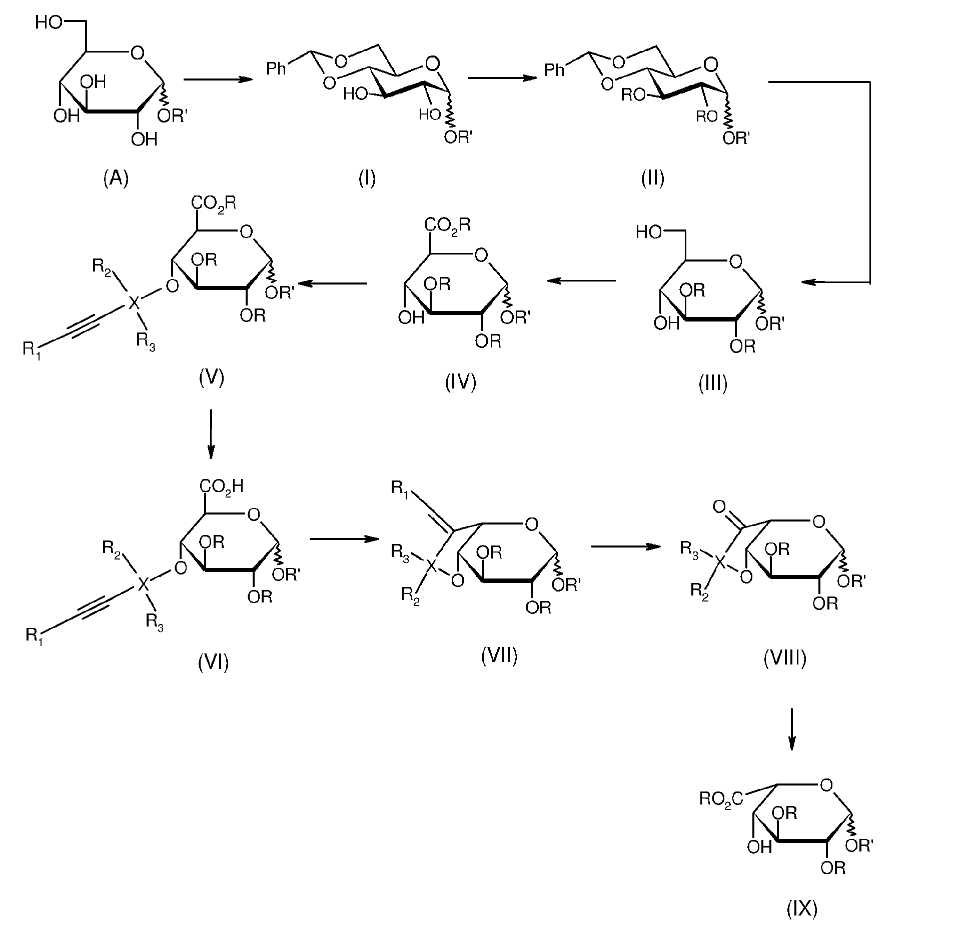

In a particular embodiment, when R' is the monosaccharide as depicted above, then the twisted bond 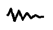 is in β position, and the scheme of the process of preparation of the compound of formula (IX) from the compound of formula (IV) may be represented as followed :

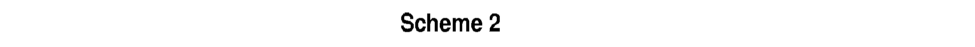

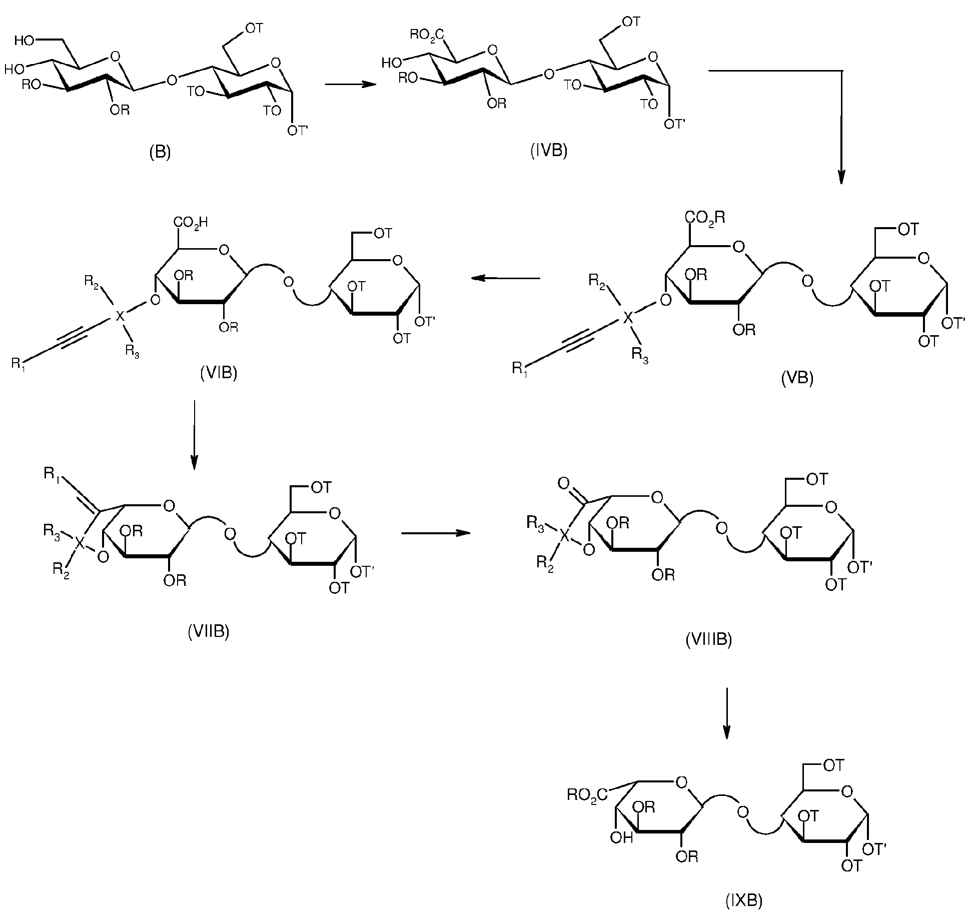

In particular, when R' is oligosaccharide, then the compound of formula (IV) may be obtained from a compound of formula (B) : 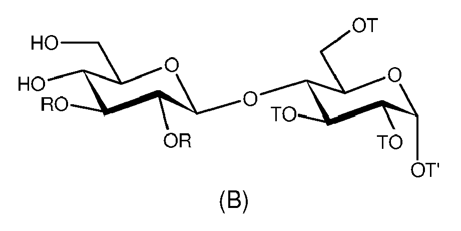 by an oxidation/esterification reaction, wherein R, T and T' are as defined above.

In the present invention, the compound (B) is prepared by application of the method as described in WO 99/25720.

According to another object, the present invention relates to a process which may allow the inversion of configuration of C5 carbon from a compound of formula (IV) 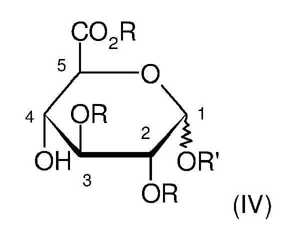 into a compound of formula (IX) : 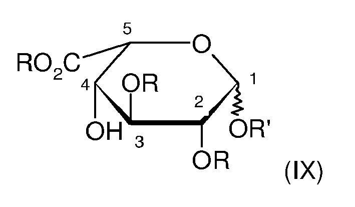 wherein 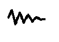 , R and R' are defined as above;
which comprises a decarboxylation/intramolecular cyclisation tandem reaction.

The inventors have advantageously found that a L-iduronic acid unit may be obtained by an easy to carry out process comprising a specific step consisting in a decarboxylation/intramolecular cyclisation tandem reaction.

The processes of the invention involve novel intermediates (V), (VI), (VII), (VIII) and (IX). In particular, the processes of the invention involve novel intermediates of formulae (VA), (VB), (VIA), (VIB), (VIIA), (VIIB), (VIIIA), (VIIIB) and (IXA), and more particularly :

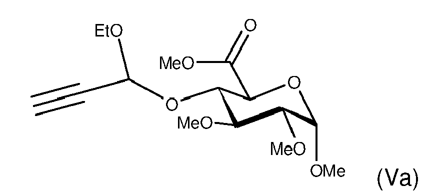

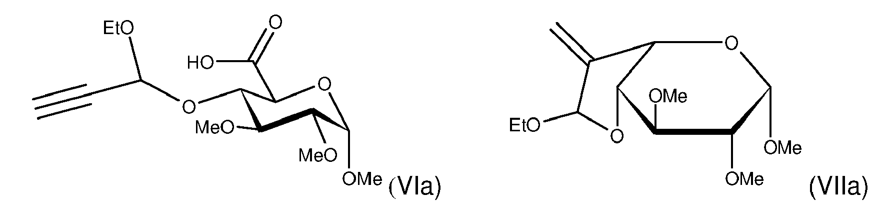

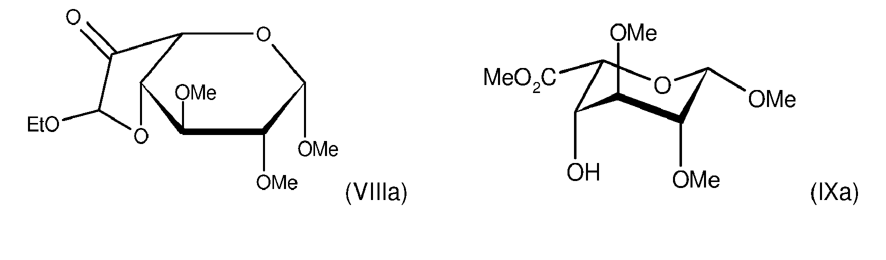

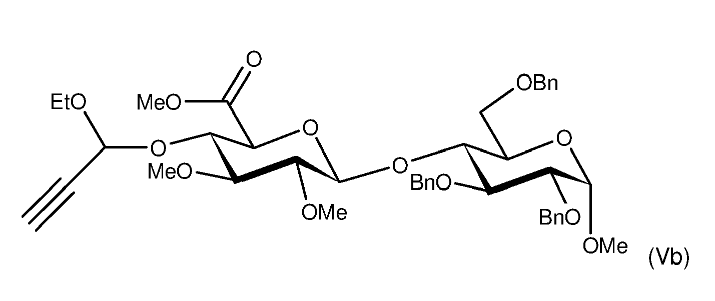

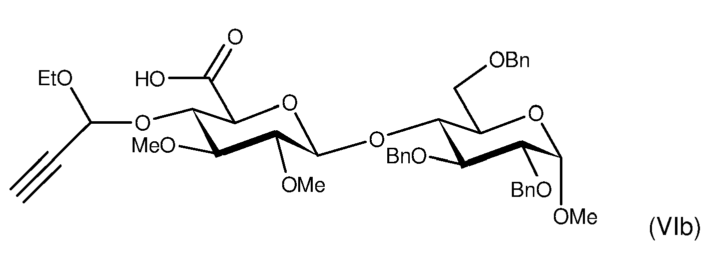

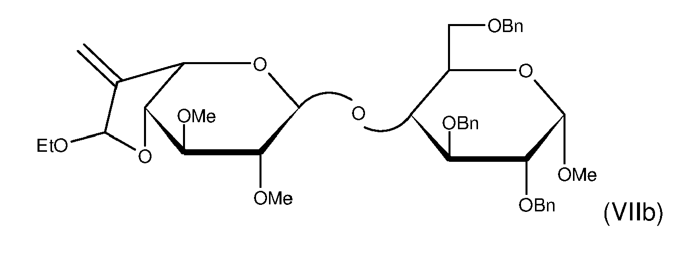

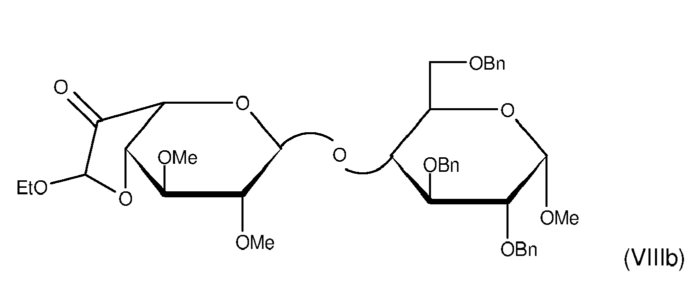

According to an other object, the present invention thus relates to a compound of formula (V) : 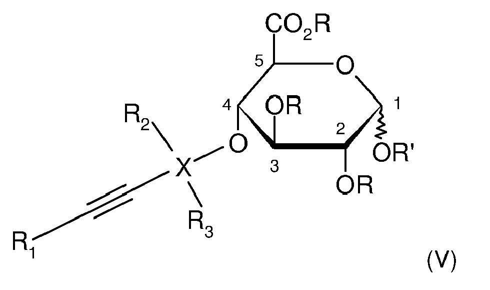 wherein :
- R is chosen from the group consisting in : alkyl, aryl and -arylalkyl;
- R' is chosen from the group consisting in : alkyl, aryl, -arylalkyl and oligosaccharide;
- R₁ represents H or alkyl;
- R₂ represents H or -O-alkyl;
- R₃ represents H or -O-alkyl;
- X represents C or Si; provided that when X represents Si, then R₃ is alkyl or when X represents C, then C is an asymmetric carbon which may possess either the (R) or the (S) configuration;
- 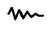 represents either α or β.

In particular, the present invention relates to compounds of formulae (VA) and (VB) above.

Among the compounds of formula (V), the following compounds may be cited : 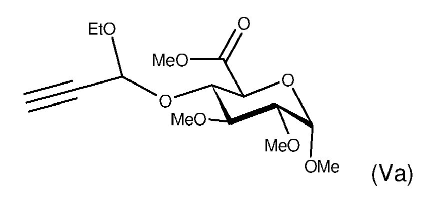 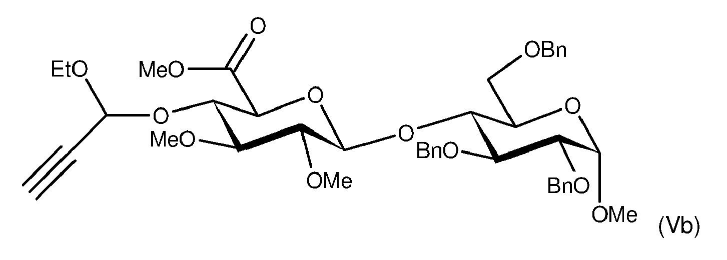 According to an other object, the present invention relates to compound of formula (VI) : 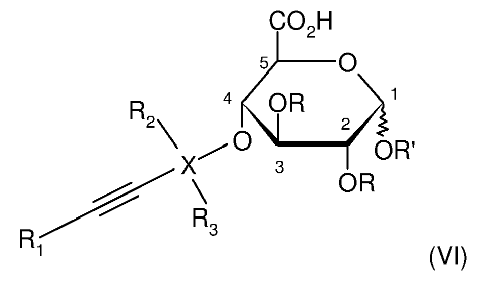 wherein 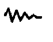 , R, R', X, R₁, R₂ and R₃ are as defined above in compound of formula (V).

In particular, the present invention relates to compounds of formulae (VIA) and (VIB) above.

Among the compounds of formula (VI), the following compounds may be cited :

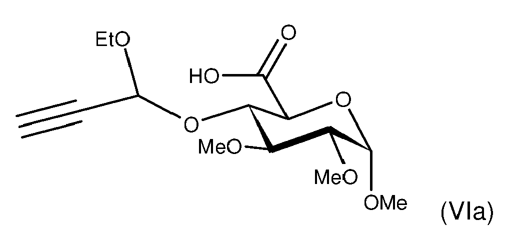

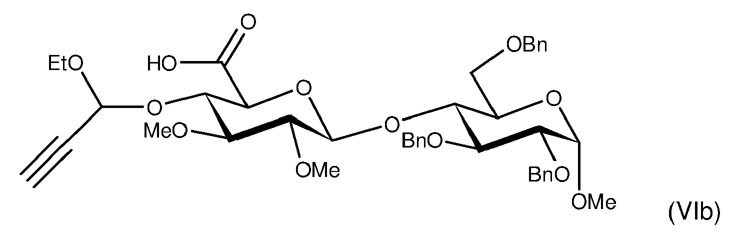

According to an other object, the present invention relates to compound of formula (VII) : 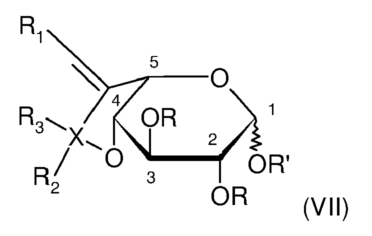 wherein 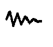 , R, R', X, R₁, R₂ and R₃ are as defined above.

In particular, the present invention relates to compounds of formulae (VIIA) and (VIIB) above.

Among the compounds of formula (VII), the following compounds may be cited :

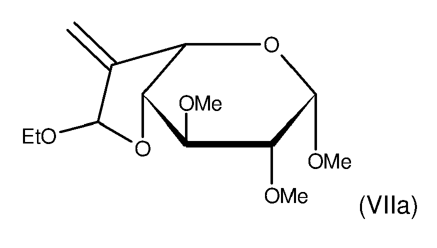

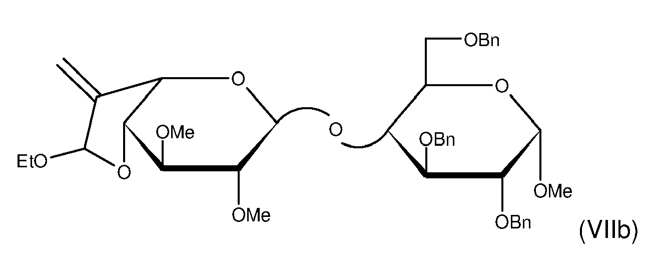

According to an other object, the present invention relates to compound of formula (VIII) : 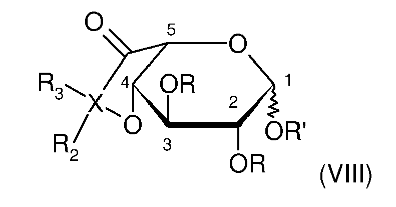 wherein 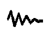 , R, R', X, R₁, R₂ and R₃ are as defined above.

In particular, the present invention relates to compounds of formulae (VIIIA) and (VIIIB) above.

Among the compounds of formula (VIII), the following compounds may be cited :

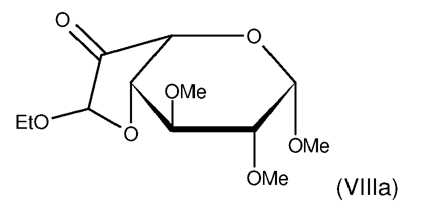

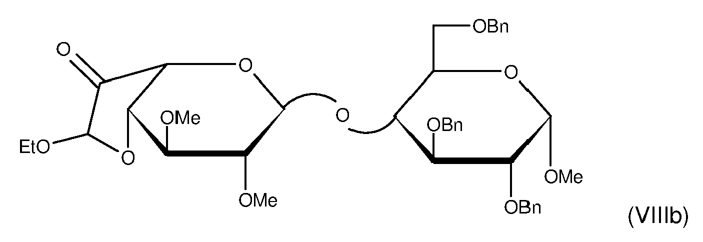

According to an other object, the present invention relates to compounds of formula (IX) : 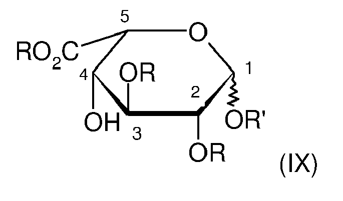 wherein 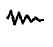 , R and R' are as defined above, except the compound of formula (IX) wherein R represents methyl and R' represents the oligosaccharide of formula : 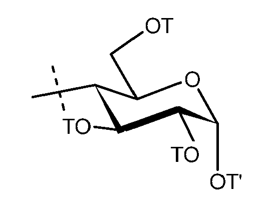 wherein T is benzyl and T' is methyl.

In particular, the present invention relates to compounds of formula (IXA) above, and more particularly the compounds of formula (IX) : 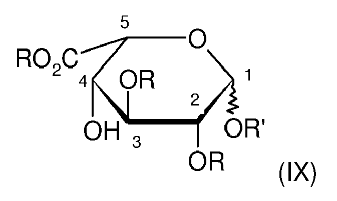 wherein 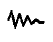 is a defined above, R and R' represent methyl, such as :

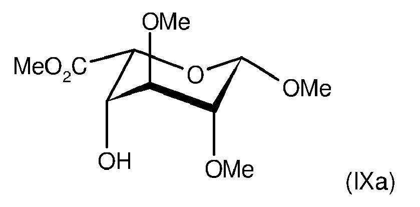

According to an aspect of the invention, the compound of formula (IX) may present either the boat conformation or the chair conformation, due to the flexibility of the L-iduronic acid.

In a particular embodiment, the compound of formula (IXA), such as (IXa) possesses a chair conformation. The inventors have found such conformation, thanks to to the coupling constants determined by NMR.

In a particular embodiment, the compound of formula (IXA), such as (IXa) possesses a chair conformation.

Idraparinux is developed in phase III trial for the prevention of thromboembolic events in patients with atrial fibrillation and for the prevention and treatment of venous thromboembolic events (VTE).

The preparation of ldraparinux is already described in Westerduin et al. (BioOrg. Med. Chem. 1994, 2, 1267-1280). EP 0 529 715 also described the synthesis of ldraparinux which comprises about forty steps. Thus, an efficient synthetic route to ldraparinux is needed.

According to another object, the present invention relates to a process of preparation of ldraparinux comprising the process of preparation of a compound of formula (IX) from a compound of formula (IV), wherein R is methyl and R' is an oligosaccharide.

In one embodiment, the present invention relates to a process of preparation of ldraparinux comprising the process of preparation of a compound of formula (IX) from a compound of formula (IV), wherein in anyone of formulae (II) to (IX), R1, R2, R3 and X are as defined above, R is methyl and R' is the monosaccharide of formula :

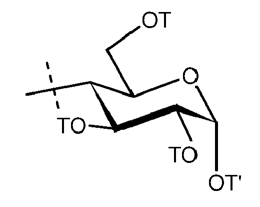

The process of preparation of Idraparinux may comprise the following steps :
1) preparation a compound of formula (IXB) wherein R is methyl, T is benzyl and T' is methyl, by the process according to the invention;
2) epimerisation of the disaccharide (IXB) so as to form disaccharide D of formula : 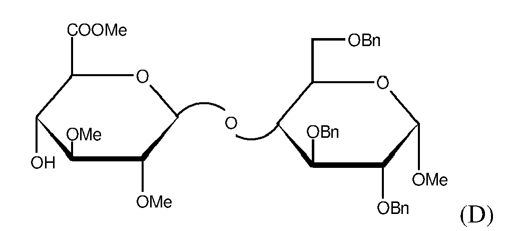
3) protection of the 4'-OH of D with a levulinoyl ester;
4) acetolysis of the disaccharide resulting from step 3), followed by preparation of the corresponding imidate;
5) coupling the disaccharide imidate resulting from step 4) with (IXB) obtainable by the process of the invention, wherein R is methyl, T is benzyl and T' is methyl, to obtain a tetrasaccharide;
6) coupling the fully protected tetrasaccharide with a monosaccharide glycosyl imidate;
7) deprotection of the protecting groups by the successive saponification and hydrogenolysis;
8) sulfation of the hydroxyl groups.

The inventors advantageously found that the process of preparation of ldraparinux comprising the decarboxylation/intramolecular cyclisation tandem reaction, which allows the inversion of configuration of C5 carbon of the compound of formula (VI), is more efficient than the processes previously described in the literature. Indeed, the process according to the invention allows advantageously a significant decrease of the number of steps and thus an improvement of the overall yield. Thus, the process of preparation of Idraparinux may be carried out in industrial scales. The inventors found an efficient process of preparation of Idraparinux.

According to another object, the present invention concerns the use of compounds of formulae (V), (VI), (VII), (VIII) and (IX), as intermediates for the preparation of ldraparinux. In particular, the present invention concerns the use of a compounds of formulae (VB), (VIB), (VIIB), (VIIIB) and (IXB), as intermediates for the preparation of ldraparinux

The invention is further illustrated but not restricted by the description in the following examples.

### Example 1 :

### Preparation of Methyl-4,6-O-benzylidene-α-D-glucopyranoside (Ia)

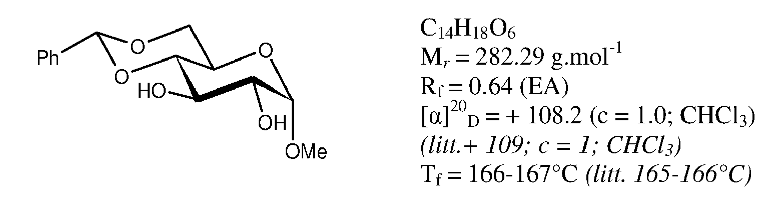

To a solution of benzaldehyde (400 mL, 3.94 mol, 5.9 eq.) was added zinc chloride (100.3 g, 0.74 mol, 1.1 eq.) under vigorous stirring. After homogenization of the solution methyl-a-D-glucopyranoside (129.6 g, 0.67 mol, 1.0 eq.) was added portionwise. After 16 hours stirring at room temperature the reaction mixture was diluted with diethyl ether (100 mL). The mixture was then poured dropwise and under vigorous stirring in a solution containing ice water (1.5 L) and hexane (350 mL). The precipitate was filtered, washed with diethyl ether (3 x 300 mL) and dried under vacuum over KOH. The product was then recrystallised from CH₂Cl₂ (720 mL) and washed with a Et₂O/CH₂Cl₂ solution (75:25, 2 x 200 mL). The filtrate was repeatedly recrystallised five times from CH₂Cl₂ to afford compound **Ia** as white crystals (136.97 g, 0.49 mol, 72%).

**¹H NMR** (CDCl₃, 250 MHz): δ 2.35 (d, J_{CH}-_{OH} = 9.2 Hz, 1 H, OH), 2.83 (d, J_{CH}-_{OH} = 2.2 Hz, 1 H, OH), 3.46 (s, 3H, -OCH₃), 3.43-3.46 (m, 1 H, H-4), 3.63 (td, J_{CH}-_{OH} = *J*_{2,3} = 9.2 Hz, *J*_{1,2} == 3.9 Hz, 1 H, H-2), 3.70-3.81 (m, 2H, H-5, H-6), 3.93 (td, J= 9.2 Hz, J_{CH}-_{OH} = 2.2 Hz, 1 H, H-3), 4.29 (m, 1 H, H-6), 4.79 (d, *J*_{1,2} = 3.9 Hz, 1 H, H-1), 5.54 (s, 1 H, Ph-CH), 7.35-7.38 (m, 3H, H_{Ar}), 7.47-7.51 (m, 2H, H_{Ar}).

¹**³C NMR** (CDCl₃, 62.9 MHz): δ 55.6 (-OCH₃), 62.5 (C-5), 69.0 (C-6), 71.1 (C-3), 72.9 (C-2), 81.0 (C-4), 99.9 (C-1), 102.0 (Ph-CH), 126.4, 128.5, 129.4, 137.1 (6xC_{Ar}).

**IR** (film) v (cm-¹): 3369 (O-H).

### Preparation of Methyl-2,3-di-O-methyl-4,6-O-benzylidene-α-D-glucopyranoside (IIa)

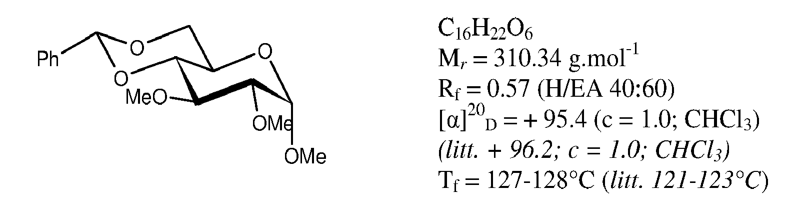

To a solution of compound la (47.60 g, 0.17 mol, 1.0 eq.) in anhydrous THF (750 mL) under an argon atmosphere and cooled to 0°C, was added portionwise sodium hydride (60%, 16.93 g, 0.42 mol, 2.5 eq.). After 20 minutes methyl iodide was added dropwise (30 mL, 0.48 mol, 2.8 eq.) and the reaction mixture was allowed to reach room temperature. After 16 hours, methanol was added portionwise (75 mL) and the solution was stirred for another 15 minutes before being concentrated. The resulting residue was dissolved in EtOAc (400 mL) and washed with water (2 x 250 mL). The organic layer was dried (MgSO₄) filtered and concentrated. The resulting solid was dissolved in diethyl ether (1000 mL), hexane was added (400 mL) and the solvent was partially evaporated at low temperature. The crystals obtained were washed with hexane and the filtrate once again partially evaporated, filtered and the precipitate washed with hexane. The combined precipitates afforded compound **IIa** as white crystals (46.10 g, 0.15 mol, 88%).

**¹H NMR** (CDCl₃, 250 MHz): δ 3.30 (dd, *J*₂₋₃ = 9.1 Hz, *J*₁₋₂ = 3.7 Hz, 1 H, H-2), 3.44 (s, 3H, - OCH₃), 3.49-3.87 (m, 4H, H-4, H-5, H-6, H-3), 3.55 (s, 3H, -OCH₃), 3.64 (s, 3H, -OCH₃), 4.28 (dd, *J*_{6-6'} = 9.1 Hz, *J*₅₋₆ = 3.7 Hz, 1 H, H-6), 4.85 (d, *J*₁₋₂ = 3.7 Hz, 1 H, H-1), 5.54 (s, 1 H, Ph-CH), 7.36-7.41 (m, 3H, H_{Ar}), 7.48-7.52 (m, 2H, H_{Ar}).

**¹³C NMR** (CDCl₃, 62.9 MHz): δ 55.4, 59.5, 61.1 (3x-OCH₃), 62.3 (C-5), 69.1 (C-6), 79.9 (C-3), 81.5 (C-4), 82.2 (C-2), 98.5 (C-1), 101.4 (Ph-CH), 126.2, 128.3, 129.0, 137.4 (6xC_{Ar}).

### Preparation of Methyl-2,3-di-O-methyl-α-D-glucopyranoside (IIIa)

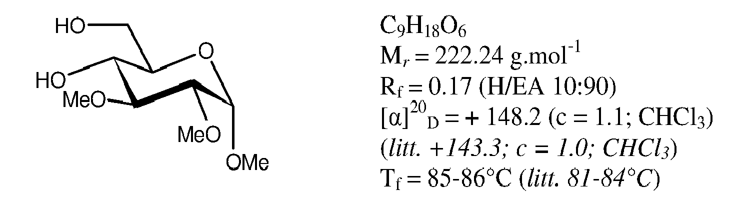

To a suspension of compound **IIa** (10.33 g, 33.29 mmol, 1.0 eq.) in methanol (150 mL) was added para-toluenesulfonic acid monohydrate (322 mg, 1.69 mmol, 0.05 eq.). After 4 hours stirring at room temperature, sodium carbonate (300 mg) was added and the reaction mixture was stirred an additional 15 minutes before filtration through a pad of Celite^{®}. Then the filtrate was concentrated and the residue obtained was dissolved in a mixture of distilled water/diethyl ether (3:1, 150 mL). The organic layer was extracted with water (2 x 50 mL) then the combined aqueous phases were concentrated and dried one night under vacuum over KOH. The resulting residue was recrystallised from toluene using petroleum ether as a co-solvent. The crystals obtained were washed with hexane and dried under vacuum over KOH to obtain compound **IIIa** as white crystals (6.63 g, 29.83 mmol, 90%).

**¹H NMR** (DMSO, 400 MHz): δ 3.03 (dd, *J*₂₋₃ = 9.3 Hz, *J*₁₋₂ = 3.5 Hz, 1 H, H-2), 3.12-3.20 (m, 2H, H-3, H-4), 3.27 (s, 3H, -OCH₃), 3.32 (s, 3H, -OCH₃), 3.30-3.33 (m, 1 H, H-5), 3.44 (s, 3H, -OCH₃), 3.40-3.46 (m, 1 H, H-6), 3.62 (ddd, *J*_{6-6'} = 11.6 Hz, *J*_{CH-OH} = 5.7 Hz, *J*₅₋₆ = 1,9 Hz, 1 H, H-6'), 4.52 (t, J = 5.7 Hz, 1 H, OH), 4.78 (d, *J*₁₋₂ = 3.5 Hz, 1 H, H-1), 5.09 (d, J_{CH}-_{OH} = 3.4 Hz, 1 H, OH).

¹³C NMR (DMSO, 100.6 MHz): δ 54.1, 57.4, 60.0 (3x-OCH₃), 60.6 (C-6), 69.5 (C-3), 72.5 (C-5), 80.9 (C-2), 82.8 (C-4), 96.4 (C-1).

**IR** (film) v (cm-¹): 3419 (O-H).

### Preparation of Methyl methyl-2,3-di-O-methyl-α-D-glucopyranosiduronate (IVa)

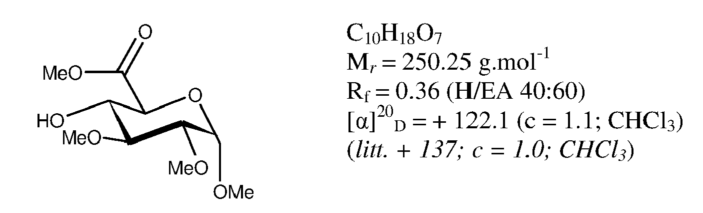

To a solution of compound **IIIa** (500 mg, 2.25 mmol, 1.0 eq.) in distilled water (15 mL) were successively added NaBr (50 mg, 0.49 mmol, 0.2 eq.) and TEMPO (7 mg, 0.05 mmol, 0.02 eq.). The reaction mixture was cooled with the aid of an ice bath then a solution of NaOCl (13% v/v, 5.2 mL, 9.1 mmol, 4.0 eq.) was added. After 5 hours stirring at 0°C ethanol was added (96% v/v, 8 mL), then the pH was reduced to 2-3 by addition of HCl (10 % v/v). The solvent was evaporated and the residue obtained was suspended in methanol, filtered in order to remove the remaining salts and washed several times with dichloromethane and methanol. The filtrate was concentrated then dissolved, under an argon atmosphere, in dry methanol (40 mL). *para*-toluenesulfonic acid (85 mg, 0.45 mmol, 0.2 eq.) was added then the reaction mixture was heated under reflux overnight. The solvent was evaporated and the residue obtained was dissolved in EtOAc (60 mL). The organic layer was washed with a 5% aqueous NaHCO₃ solution (2 x 20 mL) and with brine (1 x 20 mL). The aqueous phase was extracted with dichloromethane (3 x 20 mL). The combined organics were dried (MgSO₄), filtered and evaporated. Column chromatography (hexane/ethyl acetate 50:50) gave compound **IVa** as a colourless oil (503 mg, 2.00 mmol, 89%).

**¹H NMR** (CDCl₃, 400 MHz): δ 3.10 (d, J_{CH}-_{OH} = 3.0 Hz, 1 H, OH), 3.26 (dd, *J*₂₋₃ = 9.3 Hz, *J*₁₋₂ = 3.4 Hz, 1 H, H-2), 3.47 (s, 3H, -OCH₃), 3.49-3.52 (m, 1 H, H-3), 3.50 (s, 3H, -OCH₃), 3.62 (s, 3H, -OCH₃), 3.74 (td, J = 9.5 Hz, J_{CH}-_{OH} = 3.0 Hz, 1 H, H-4), 3.82 (s, 3H, -OCH₃), 4.14 (d, *J*₄₋₅ = 9.6 Hz, 1 H, H-5), 4.91 (d, *J*₁₋₂ = 3.4 Hz, 1 H, H-1).

**¹³C NMR** (CDCl₃, 100.6 MHz): δ 52.9, 56.0, 59.1, 61.3 (4x-OCH₃), 70.6 (C-5), 71.7 (C-4), 80.9 (C-2), 81.8 (C-3), 98.1 (C-1), 170.9 (C=O).

**IR** (film) v (cm-¹): 3475 (O-H), 1750 (C=O).

### Preparation of Methyl methyl-4-O-(1'-ethoxy-2'-propyn-1'-yl)-2,3-di-O-methyl-α-D-glucopyranosiduronate (Va)

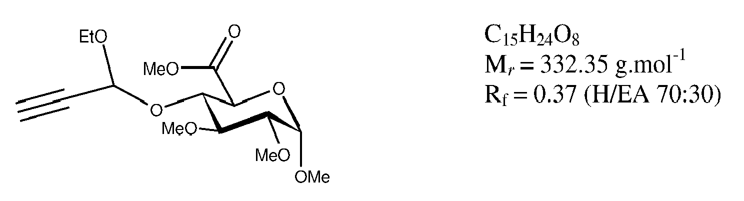

To a solution of compound **lVa** (4.56 g, 18.21 mmol, 1.0 eq.) in chloroform (stabilised with amylene, 200 mL) were added, under an argon atmosphere, P₂O₅ (5.31 g, 36.29 mmol, 2.0 eq.) and propargylaldehyde diethylacetal (5.2 mL, 36.27 mmol, 2.0 eq.), then the reaction mixture was heated at 60°C. After 4 hours stirring, the reaction mixture was filtered through a pad of Celite^{®} then the solvent was removed under vacuum. The crude mixture was suspended in EtOAc (300 mL), washed with a 5% NaHCO₃ aqueous solution (1 x 30 mL) and brine (1 x 30 mL). The organic layer was dried (MgSO₄), filtered, and evaporated. Column chromatography (gradient hexane/ethyl acetate 80:20 - 20:80) afforded compound **Va** as a colourless oil (4.07 g, 12.24 mmol, 67%) in a diastereomeric mixture (64:36) (the relative composition of the mixture was determined by ¹H NMR from integrations of protons EtO-*CH*), along with some unreacted compound **lVa** (1.17 g, 4.68 mmol, 26%).

**¹H NMR** (CDCl₃, 400 MHz): δ 1.18-1.25 (m, 3H, -OCH₂*CH*₃) (diastereomeric mixture), 2.56 (m, 1 H, H-C≡C-) (mixture), 3.26-3.31 (m, 1 H, H-2) (mixture), 3.43 (s, 3H, -OCH₃) (major), 3.44 (s, 3H, -OCH₃) (minor), 3.50 (s, 3H, -OCH₃) (mixture), 3.59 (s, 3H, -OCH₃) (minor), 3.62 (s, 3H, -OCH₃) (major), 3.47-3.62 (m, 2H, H-3, -OC*H*ₐH_{b}CH₃) (mixture), 3.65-3.73 (m, 1 H, -OCHₐH_{b}CH₃) (mixture), 3.78 (s, 3H, -OCH₃) (major), 3.80 (s, 3H, -OCH₃) (minor), 3.78-3.86 (m, 1 H, H-4) (mixture), 4.15 (d, *J*₄₋₅ = 10.0 Hz, 1 H, H-5) (major), 4.18 (d, J₄-₅ = 10.0 Hz, 1 H, H-5) (minor), 4.86-4.88 (m, 1 H, H-1) (mixture), 5.35 (d, J = 1.7 Hz, 1 H, EtO-*CH*) (minor), 5.58 (d, *J*= 1.7 Hz, 1 H, EtO-CH) (major).

**¹³C NMR** (CDCl₃, 100.6 MHz): 15.0 (-OCH₂CH₃₎ (mixture), 52.6 (-OCH₃) (major), 52.7 (-OCH₃) (minor), 55.8 (-OCH₃) (mixture), 59.2 (-OCH₃) (major), 59.3 (-OCH₃) (minor), 60.4 (-OCH₂CH₃) (major), 61.3 (-OCH₂CH₃) (minor), 61.4 (-OCH₃) (mixture), 70.1 (C-5) (minor), 70.2 (C-5) (major), 74.0 (H-*C*≡C-) (major), 74.2 (H-*C*≡C-) (minor), 76.4 (C-4) (minor), 76.7 (C-4) (major), 78.6 (H-C≡*C*-) (minor), 78.9 (H-C≡*C*-) (major), 81.5 (C-2) (major), 81.8 (C-2) (minor), 81.9 (C-3) (minor), 82.9 (C-3) (major), 92.6 (EtO-CH) (mixture), 97.9 (C-1) (minor), 98.0 (C-1) (major), 169.6 (C=O) (major), 169.9 (C=O) (minor).

**Elemental analysis:** Calculated: C: 54.21 ; H: 7.28. Found: C: 54.17 ; H: 7.13.

**ESI-MS** (pos. mode): m/z= 355 [M+Na]⁺.

**IR** (film) v (cm-¹): 1752 (C≡O), 3266 (≡C-H).

### Preparation of 4-O-(l'-ethoxy-2'-propyn-1'-yl)-1,2,3-tri-O-methyl-α- D-gluco-pyranosiduronic acid (VIa)

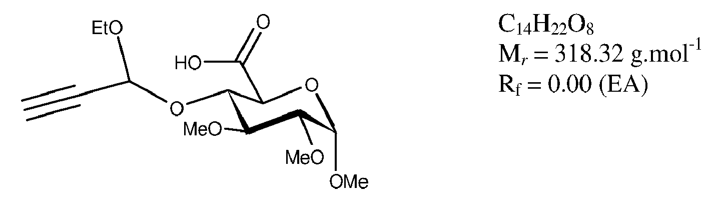

To a solution of compound **Va** (1.12 g, 3.37 mmol, 1.0 eq.) in EtOH/H₂O (3:1, 100 mL) was added sodium hydroxide (156 mg, 3.90 mmol, 1.3 eq.). After 5 hours stirring at room temperature the solvent was evaporated. The residue obtained was dissolved in water (50 mL). The pH of the aqueous layer was reduced to 2-3 with a 5% citric acid aqueous solution, then the layer was saturated with sodium chloride before extraction with dichloromethane (10 x 20 mL). If necessary the pH was adjusted by addition of more citric acid aqueous solution. The combined organics were dried (MgSO₄), filtered and removed under vacuum. Compound **Via** was obtained without further purification as a colourless oil (1.020 g, 3.20 mmol, 95%), in a mixture of diastereomers (75:25) (the relative composition of the mixture was determined by ¹H NMR from integrations of protons EtO-*CH*.

**¹H NMR** (CDCl₃, 400 MHz): δ 1.16-1.24 (m, 3H, -OCH₂*CH*₃) (diastereomeric mixture), 2.59 (d, J = 1.6 Hz, 1 H, H-C≡C-) (major), 2.62 (s I, 1 H, H-C≡C-) (minor), 3.25-3.33 (m, 1 H, H-2), 3.44 (s, 3H, -OCH₃) (mixture), 3.51 (s, 3H, -OCH₃) (mixture), 3.62 (s, 3H, -OCH₃) (mixture), 3.54-3.62 (m, 2H, H-3, -OCHₐH_{b}CH₃) (mixture), 3.68-3.77 (m, 1 H, -OCHₐ*H*_{b}CH₃) (mixture), 3.81-3.87 (m, 1 H, H-4) (mixture), 4.13-4.18 (m, 1 H, H-5) (mixture), 4.88-4.90 (m, 1 H, H-1), 5.45 (s I, 1 H, EtO-*CH*) (minor), 5.63 (d, *J* = 1.6 Hz, 1 H, EtO-*CH*(major).

**¹³C NMR** (CDCl₃, 100.6 MHz): δ 14.9 (-OCH₂*C*H₃) (mixture), 55.9 (-OCH₃) (mixture), 59.2 (-OCH₃) (minor), 59.3 (-OCH₃) (major), 60.7 (-OCH₂CH₃) (mixture), 61.2 (-OCH₃) (minor), 61.3 (-OCH₃) (major), 70.1 (C-5) (mixture), 74.3 (H-*C*≡C-) (major), 74.8 (H-*C*≡C-) (minor), 75.7 (C-4) (minor), 76.4 (C-4) (major), 78.5 (H-C≡*C*-) (minor), 78.8 (H-C≡*C*-) (major), 81.4 (C-2) (major), 81.7 (C-2) (minor), 81.8 (C-3) (minor), 82.9 (C-3) (major), 92.5 (EtO-CH) (mixture), 97.8 (C-1) (minor), 98.0 (C-1) (major), 173.8 (C=O) (major), 174.0 (C=O) (minor).

**ESI-MS** (pos. mode): m/z= 341 [M+Na]⁺.

**IR** (film) v (cm⁻¹): 1751 (C₌O), 3268 (≡C-H).

### Preparation of Methyl-4,7-anhydro-6-deoxy-6-methylene-7-ethoxy-2,3-di-O-methyl-α-L-ido-heptopyranoside (VIIa)

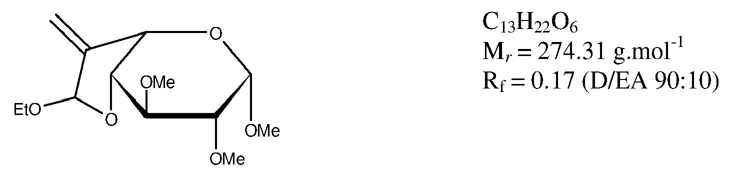

To a solution of compound **VIa** (1.89 g, 5.92 mmol, 1.0 eq.) in anhydrous THF (40 mL) and cooled to 0°C, were added IBCF (0.84 mL, 6.48 mmol, 1.1 eq.) and *N-*methylmorpholine (0.72 mL, 6.55 mmol, 1.1 eq.). After 20 minutes stirring the flask was covered with aluminium foil, 2-mercaptopyridine N oxide sodium salt (1.77 g, 11.80 mmol, 2.0 eq.) was added and the reaction mixture was stirred at ambient temperature. After 2 hours, anhydrous THF (80 mL) then tert-butylthiol (0.28 mL, 2.61 mmol, 1.6 eq.) were added. The aluminium foil was removed and the reaction mixture was irradiated and heated 30 minutes with a UV lamp (300W). The thiol excess was neutralized with a NaOCl aqueous solution (13% v/v, 10 mL). The reaction mixture was concentrated then dissolved in EtOAc (100 mL), washed successively with a 5% NaHCO₃ aqueous solution (2 x 15 mL), a 5 % citric acid aqueous solution (1 x 15 mL) and brine (1 x 25 mL), then the aqueous layer was extracted with dichloromethane (2 x 20 mL). The combined organics were dried (MgSO₄), filtered and concentrated. Column chromatography (gradient dichloromethane/ethyl acetate 95:5 ― 75:25) afforded compound **VIIa** as a colourless oil (218 mg, 0.79 mmol, 48%), in a mixture of diastereomers (67:33) (the relative composition of the mixture was determined by ¹H NMR from integrations of protons H-2).

**¹H NMR** (CDCl₃, 400 MHz): δ 1.23 (t, J= 7.1 Hz, 3H, -OCH₂*CH*₃) (diastereomeric mixture), 3.13 (dd, *J*₂₋₃ = 9.6 Hz, *J*₁₋₂ = 3.0 Hz, 1 H, H-2) (minor), 3.30 (dd, *J*₂₋₃ = 5.0 Hz, *J*₁₋₂ = 1.6 Hz, 1 H, H-2) (major), 3.41 (s, 3H, -OCH₃) (minor), 3.47 (s, 3H, -OCH₃) (major), 3.50 (s, 3H, -OCH₃) (mixture), 3.53 (s, 3H, -OCH₃) (major), 3.55-3.61 (m, 1H, -OC*H*ₐH_{b}CH₃) (mixture), 3.72 (dd, *J*₂₋₃ = 5.0 Hz, *J*₃₋₄ = 2.8 Hz, 1 H, H-3) (major), 3.78-3.90 (m, 1H, - OCHₐH_{b}CH₃) (mixture), 3.93-4.07 (m, 2H, H-3, H-4) (mixture), 4.59 (d I, *J*₄₋₅ = 4.0 Hz, 1 H, H-5) (major), 4.62 (d, *J*₁₋₂ = 1.7 Hz, 1 H, H-1) (major), (td, *J*₄₋₅ = 7.9 Hz, J = 2.6 Hz, 1 H, H-5), (minor), 4.79 (d, *J*₁₋₂ = 3.0 Hz, 1H, H-1) (minor), 5.35-5.57 (m, 3H, H-7, -C₌CH₂) (mixture).

**¹³C NMR** (CDCl₃, 100.6 MHz): δ 15.3 (-OCH₂CH₃) (minor), 15.4 (-OCH₂CH₃) (major), 56.5 (-OCH₃) (minor), 56.8 (-OCH₃) (major), 58.6 (-OCH₃) (major), 59.1 (-OCH₃) (minor), 59.9 (-OCH₃) (major), 60.3 (-OCH₃) (minor), 63.3 (-OCH₂CH₃) (minor), 63.8 (-OCH₂CH₃) (major), 74.2 (C-5) (minor), 74.7 (C-5) (major), 76.4 (C-3) (major), 77.0 (C-4) (major), 77.7 (C-2) (major), 79.0 (C-3) (minor), 79.7 (C-4) (minor), 80.1 (C-2) (minor), 99.3 (C-1) (major), 99.5 (C-1) (minor), 102.2 (C-7) (major), 103.0 (C-7) (minor), 111.6 (-C₌*C*H₂) (minor), 115.2 (-C=CH₂) (major), 147.4 (C-6) (minor), 148.1 (C-6) (major).

**ESI-MS** (pos. mode): m/z= 297 [M+Na]⁺.

### Preparation of Methyl-4,7-anhydro-7-ethoxy-2,3-di-O-methyl-α-L-ido-hepto-pyranosid-6-ulose (VIIIa)

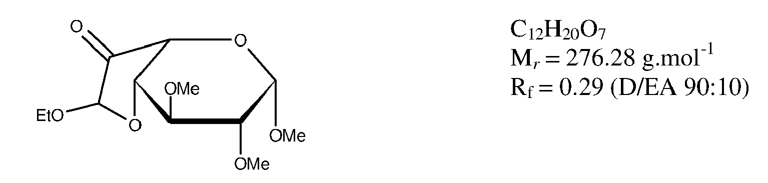

Through a solution of compound **VIIa** (449 mg, 1.64 mmol, 1.0 eq.) in anhydrous dichloromethane (10 mL), under an argon atmosphere and cooled to -78°C, was bubbled ozone (0.2 L/min, 110 V). When the solution had turned dark blue, oxygen was bubbled through in order to remove the excess ozone. When the solution became colorless dimethylsulfide (5 drops) was added and the solution was brought to room temperature. After 1h15 the reaction mixture was concentrated. Column chromatography (gradient dichloromethane/ethyl acetate 95:5 - 80:20) afforded compound **VIIIa** as a white solid (364 mg, 1.32 mmol, 80%), in a mixture of diastereomers (79:21) (the relative composition of the mixture was determined by ¹H NMR from integrations of protons H-2).

**¹H NMR** (CDCl₃, 400 MHz): δ 1.24-1.28 (m, 3H, -OCH₂C*H*₃) (diastereomeric mixture), 3.10 (dd, *J*₂₋₃ = 10.2 Hz, *J*_{*1-*2} = 2.9 Hz, 1 H, H-2) (minor), 3.17 (dd, *J*₂₋₃ = 9.4 Hz, J₁₋₂ = 2.8 Hz, 1 H, H-2) (major), 3.38 (s, 3H, -OCH₃) (major), 3.42 (s, 3H, -OCH₃) (minor), 3.50 (s, 3H, - OCH₃) (mixture), 3.63 (s, 3H, -OCH₃) (major), 3.66 (s, 3H, -OCH₃) (minor), 3.48-3.73 (m, 3H, H-3 major, -OC*H*ₐH_{b}CH₃ major, -OCHₐH_{b}CH₃ minor), 3.77-3.95 (m, 2H, -OCHₐH_{b}CH₃ minor, -OCHₐ*H*_{b}CH₃ major), 4.07 (dd, *J*₂₋₃ = 10.2 Hz, *J*₃₋₄ = 7.7 Hz, 1 H, H-3) (minor), 4.34 (d, *J*₄₋₅ = 9.1 Hz, 1 H, H-5) (minor), 4.39-4.44 (m, 2H, H-4 minor, H-5 major), 4.50 (dd, *J*₃₋₄ = 9.5 Hz, *J*₄₋₅ = 6.2 Hz, 1 H, H-4) (major), 4.76 (d, *J*₁₋₂ = 2.8 Hz, 1 H, H-1) (major), 4.79 (d, *J*₁₋₂ = 2.9 Hz, 1 H, H-1) (minor), 4.89 (d, J = 1,1 Hz, 1 H, H-7) (minor), 4.93 (s I, 1 H, H-7) (major).

**¹³C NMR** (CDCl₃, 100.6 MHz): δ 15.1 (-OCH₂CH₃) (minor), 15.2 (-OCH₂CH₃) (major), 56.7 (-OCH₃) (minor), 57.2 (-OCH₃) (major), 59.3 (-OCH₃) (mixture), 59.8 (-OCH₃) (major), 60.6 (-OCH₃) (minor), 65.0 (-OCH₂CH₃) (minor), 65.5 (-OCH₂CH₃) (major), 70.2 (C-5) (major), 72.4 (C-5) (minor), 75.9 (C-4) (major), 79.2 (C-4) (minor), 79.4 (C-3) (major), 79.8 (C-2 major, C-3 minor), 80.2 (C-2) (minor), 96.1 (C-7) (major), 97.2 (C-7) (minor), 98.7 (C-1) (major), 99.0 (C-1) (minor), 205.3 (C-6) (minor), 205.6 (C-6) (major).

**IR** (film) v (cm-¹): 1783 (C=O).

**ESI-MS** (pos. mode): m/z= 299 [M+Na]⁺, 331 [M+Na+MeOH]⁺.

### Preparation of Methyl methyl-2,3-di-O-methyl-α-L-idopyranosiduronate (IXa)

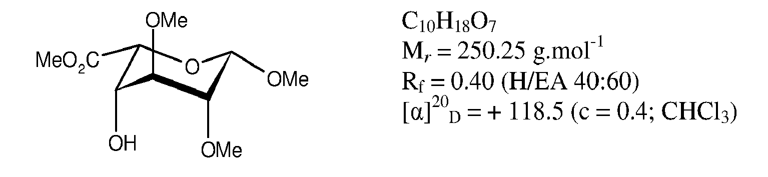

To a solution of compound **Villa** (50 mg, 0.18 mmol, 1.0 eq.) in dichloromethane (3 mL), under an argon atmosphere and cooled to 0°C, were added *m*-CPBA (77%, 120 mg, 0.54 mmol, 3.0 eq.) and NaHCO₃ (20 mg, 0.23 mmol, 1.3 eq.). After 3 hours stirring the solvent was removed under vacuum. The resulting residue was dissolved in EtOAc (30 mL), extracted with distilled water (2 x 10 mL), and the aqueous phase was concentrated. The crude mixture was dissoved in methanol (10 mL), para-toluenesulfonic acid monohydrate was added (4 mg, 0.02 mmol, 0.1 eq.) then the reaction mixture was heated to reflux and the reaction monitored by ¹H NMR in deuterated methanol. After 8 hours the solvent was evaporated. The residue obtained was dissolved in DMF (5 mL) then triethylamine (28 µL, 0.20 mmol, 1.1 eq.) and methyl iodide (56 µL, 0.90 mmol, 5 eq.) were added. After 3h30 the reaction mixture was concentrated, dissolved in EtOAc (30 mL) and the organic phase was washed with a 5% NaHC0₃ aqueous solution (2 x 10 mL), a 5% citric acid aqueous solution (2 x 10 mL) and brine (1 x 10 mL). The aqueous phase was extracted with dichloromethane (5 x 10 mL) and the combined organics were dried (MgSO₄), filtered and concentrated. Column chromatography (dichloromethane/ethyl acetate 85:15) afforded compound **XIa** as a colourless oil (25 mg, 0.10 mmol, 56%).

**¹H NMR** (CDCl₃, 400 MHz): δ 3.41 (d I, *J*₂₋₃ = 3.5 Hz, 1 H, H-2), 3.47 (s, 3H, -OCH₃), 3.56 (s, 3H, -OCH₃), 3.57 (s, 3H, -OCH₃), 3.69 (t, *J*₂₋₃ = *J*₃₋₄ = 3.5 Hz, 1 H, H-3), 3.75-3.78 (m, 1 H, OH), 3.80 (s, 3H, -OCH₃), 3.97 (m, 1 H, H-4), 4.42 (d, *J*₄₋₅ = 1.6 Hz, 1 H, H-5), 4.61 (d, *J*₁₋₂ = 0.9 Hz, 1 H, H-1).

**¹³C NMR** (CDCl₃, 100.6 MHz): δ 52.4, 57.5, 58.4, 60.8 (4x-OCH₃), 67.7 (C-4), 74.8 (C-5), 77.2 (C-2), 77.5 (C-3), 100.9 (C-1), 169.6 (C=O).

**Elemental analysis:** Calculated: C: 48.00 ; H: 7.25. Found: C: 47.62 ; H: 7.15.

**ESI-MS** (pos. mode): m/z= 272 [M+Na]⁺.

**IR** (film) v (cm-¹): 3491 (O-H), 1765 (C=O).

### Example 2 :

### Preparation of Methyl-2,3,6-tri-O-benzyl-4-O(2',3'-di-O-methyl-β-D-glucopyranosyl-uronate)-α-D-glucopyranoside (IVb)

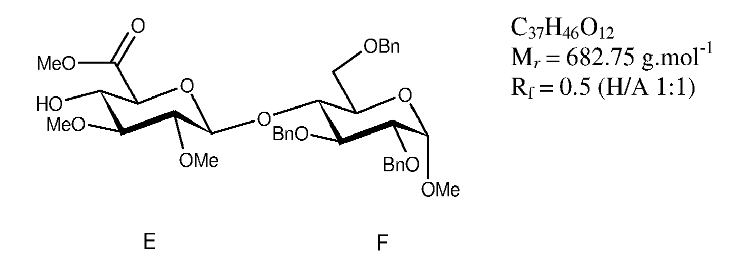

To a solution of the compound of formula 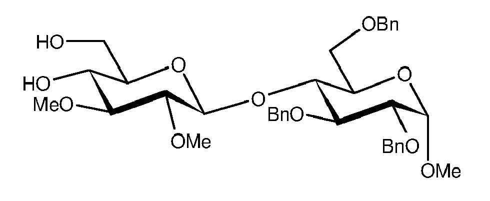 (3.279 g, 5.00 mmol, 1.0 eq.) in a water/acetonitrile mixture (1:1, 300 mL) were added NaBr (105 mg, 1.02 mmol, 0.2 eq.) and TEMPO (33 mg, 0.21 mmol, 0.04 eq.). The reaction mixture was cooled with the aid of an ice bath then a solution of NaOCl (13% v/v, 11.5 mL, 20.08 mmol, 4.0 eq.) was added. After 3 hours stirring at 0°C, NaOCl was added anew (13% v/v, 11.5 mL, 20.08 mmol, 4.0 eq.). After two more hours ethanol was added (96% v/v, 20 mL), then the pH was reduced to 2-3 by addition of HCl (10% v/v). The solvent was evaporated and the residue obtained was suspended in DMF (40 mL) then triethylamine (2.8 mL, 2.032 g, 20.0 mmol, 4.0 eq.) and methyl iodide (6.2 mL, 14.136 g, 99.6 mmol, 20.0 eq.) were added. After 4 hours stirring at room temperature the solvent was evaporated and the residue obtained was dissolved in EtOAc (200 mL). The organic layer was washed with a 5% citric acid aqueous solution (1 x 20 mL) and brine (1 x 20 mL). The aqueous layer was extracted with dichloromethane (2 x 20 mL). The combined organics were dried (MgSO₄), filtered and evaporated. The residue obtained was dissolved in DMF (20 mL), then triethylamine (1.4 mL, 1.016 g, 10.0 mmol, 2.0 eq.) and methyl iodide (3.1 mL, 7.068 g, 49.8 mmol, 10.0 eq) were added. After 60 hours stirring at room temperature the solvent was evaporated and the residue obtained was dissolved in EtOAc (200 mL). The organic layer was washed with a 5% citric acid aqueous solution (2 x 20 mL) and brine (1 x 20 mL). The organic layer was dried (MgSO₄), filtered and evaporated. Column chromatography (gradient hexane/ethyl acetate 80:20 - 50:50) gave compound **lVb** as a colourless oil which was dissolved in a diethyl ether/hexane mixture and evaporated at room temperature to afford a white solid (2.500 g, 3.66 mmol, 73%).

**¹H NMR** (CDCl₃, 400 MHz): δ 2.92 (d, 1 H), 2.93-2.98 (m, 2H), 3.41 (s, 3H), 3.48-3.76 (m, 5H), 3.51 (s, 3H), 3.60 (s, 3H), 3.63 (s, 3H), 3.87-3.98 (m, 3H), 4.36-4.41 (m, 1 H), 4.49-5.08 (m, 6H), 4.61 (d, 1 H), 7.24-7.42 (m, 15H).

**Elemental analysis:** Calculated: C: 65.09 ; H: 6.79. Found: C: 65.29 ; H: 6.96.

**ESI-MS** (pos. mode): m/z = 705 [M+Na]⁺.

### Preparation of Methyl-2,3,6-tri-O-benzyl-4-O(4'-O-(1"-ethoxy-2"-propyn-1"-yl)-2',3'-di-O-methyl-β-D-glucopyranosyluronate)-α-D-glucopyranoside (Vb)

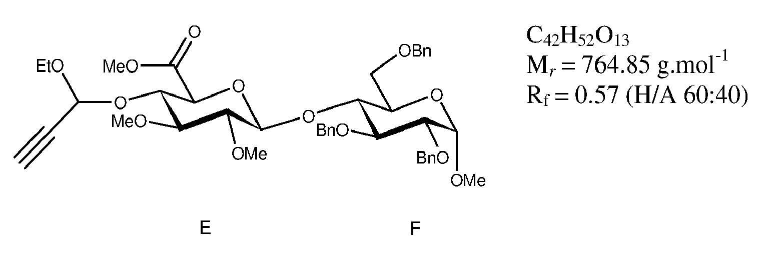

To a solution of compound **lVb** (385 mg, 0.56 mmol, 1.0 eq.) in chloroform (stabilised with amylene, 30 mL) were added, under an argon atmosphere, P₂O₅ (410 mg, 2.80 mmol, 5.0 eq.) and propargylaldehyde diethylacetal (0.4 mL, 2.79 mmol, 5.0 eq.), then the reaction mixture was heated at reflux. After 5 hours stirring, the reaction mixture was filtered through a pad of Celite^{®} then the solvent was removed under vacuum. The crude mixture was suspended in EtOAc (60 mL), washed with a 5% NaHCO₃ aqueous solution (1 x 15 mL) and brine (1 x 15 mL). The organic layer was dried (MgSO₄), filtered, and evaporated. Column chromatography (gradient hexane/ethyl acetate 90:10 ― 70:30) afforded compound Vb as a colourless oil (275 mg, 0.36 mmol, 64%) in a diastereomeric mixture (64:36).

**¹H NMR** (CDCl₃, 250 MHz): δ 1.17-1.27 (m, 3H), 2.55 (d, 0.36H), 2.57 (d, 0.64H), 2.92-3.08 (m, 2H), 3.38 (s, 3H), 3.49 (s, 1.92H), 3.50 (s, 1.08H), 3.57 (s, 1.08H), 3.59 (s, 1.92H), 3.60 (s, 1.92H), 3.62 (s, 1.08H), 3.44-3.97 (m, 10H), 4.35 (t, 1 H), 4.46-4.76 (m, 6H), 5.03 (d, 1 H), 5.32 (d, 0.36H), 5.56 (d, 0.64H), 7.21-7.42 (m, 15H).

**Elemental analysis:** Calculated: C: 65.95 ; H: 6.85. Found: C: 65.92 ; H: 6.75.

**ESI-MS** (pos. mode): m/z= 787 [M+Na]⁺.

### Preparation of Methyl-2,3,6-tri-O-benzyl-4-O(4'-O-(1"-ethoxy-2"-propyn-1"-yl)-1',2',3'-tri-O-methyl-α-D-glucopyranosiduronic acid)-a-D-glucopyranoside (Vlb)

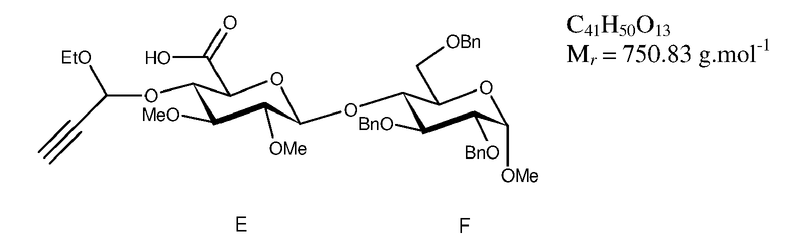

To a solution of compound **Vb** (1.02 g, 1.33 mmol, 1.0 eq.) in EtOH/H₂O (1:1, 100 mL) was added sodium hydroxide (82 mg, 2.05 mmol, 1.5 eq.). After 3 hours stirring at room temperature sodium hydroxide was added anew (27 mg, 0.68 mmol, 0.5 eq.). After an additional hour stirring the solvent was evaporated. The residue obtained was dissolved in water (40 mL). The pH of the aqueous layer was reduced to 2-3 with a 10% HCl aqueous solution then the layer was saturated with sodium chloride before extraction with dichloromethane (3 x 20 mL). The combined organics were dried (MgSO₄), filtered and removed under vacuum. Compound **VIb** was obtained without further purification as a white solid (930 mg, 1.24 mmol, 93%), in a diastereomeric mixture (63:37).

**¹H NMR** (CDCl₃, 400 MHz): δ 1.17-1.28 (m, 3H), 2.65 (d, 0.63H), 2.68 (d, 0.37H), 2.90-3.09 (m, 2H), 3.37 (s, 3H), 3.46 (s, 1.11 H), 3.57 (s, 1.89H), 3.58 (s, 1.11 H), 3.60 (s, 1.89H), 3.42-3.90 (m, 10H), 4.29 (d, 1 H), 4.46-4.97 (m, 7H), 5.44 (d, 0.37H), 5.61 (d, 0.63H), 7.28-7.44 (m, 15H).

**ESI-MS** (pos. mode): m/z = 773 [M+Na]⁺ , 795 [M-H+2Na]⁺ .

**ESI-MS** (neg. mode): m/z = 749 [M-H]-.

### Preparation of Methyl-2,3,6-tri-O-benzyl-4-O(4',7'-anhydro-6'-deoxy-6'-methylene-7'-ethoxy-2',3'-di-O-methyl-α-L-ido-heptopyranosyl)-α-D-glucopyranoside (Vllb)

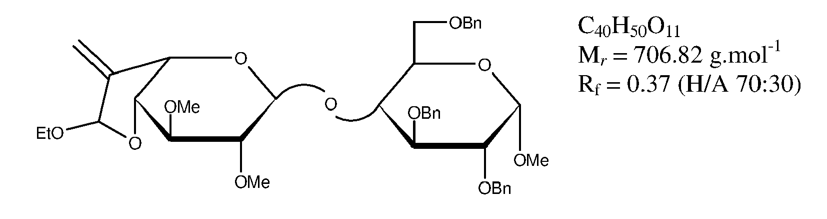

To a solution of compound **VIb** (647 mg, 0.86 mmol, 1.0 eq.) in anhydrous THF (20 mL) and cooled to 0°C, were added IBCF (0.11 mL, 0.85 mmol, 1.0 eq.) and N-methylmorpholine (0.10 mL, 0.91 mmol, 1.1 eq.). After 10 minutes stirring, the flask was covered with aluminium foil, 2-mercaptopyridine *N*-oxide sodium salt (512 mg, 3.43 mmol, 4.0 eq.) was added and the reaction mixture was stirred at ambient temperature. After 20 minutes anhydrous THF (100 mL) then tert-butylthiol (0.18 mL, 1.68 mmol, 2.0 eq.) were added. The aluminium foil was removed and the reaction mixture was irradiated and heated 15 minutes with a UV lamp (300W). The thiol excess was neutralized with a NaOCl aqueous solution (13%, 10 mL). The reaction mixture was concentrated then dissolved in EtOAc (100 mL), washed successively with a 5% NaHCO₃ aqueous solution (1 x 15 mL), a 5 % citric acid aqueous solution (1 x 15 mL) and brine (1 x 15 mL), then the aqueous layer was extracted with dichloromethane (2 x 20 mL). The combined organics were dried (MgSO₄), filtered and concentrated. Column chromatography (gradient hexane/ethyl acetate 90 :10 - 70:30) afforded compound **Vllb** as a colourless oil (251 mg, 0.36 mmol, 42%) in a mixture of diastereomers (61:39).

**¹H NMR** (CDCl₃, 250 MHz): δ 1.18-1.29 (m, 3H), 2.90-3.07 (m, 1 H), 3.37 (s, 1.17H), 3.38 (s, 1.83H), 3.46 (s, 3H), 3.54 (s, 1.83H), 3.60 (s, 1.17H), 3.29-4.00 (m, 10H), 4.11-4.26 (m, 1 H), 4.50-4.96 (m, 8H), 5.09-5.48 (m, 3H), 7.23-7.39 (m, 15H).

**ESI-MS** (pos. mode): m/z= 720 [M+Na]⁺.

### Preparation of Methyl-2,3,6-tri-O-benzyl-4-O(4',7'-anhydro-7'-ethoxy-2',3'-di-O. methyl-α-L-ido-heptopyranosid-6'-ulosyl)-α-D-glucopyranoside (Vlllb)

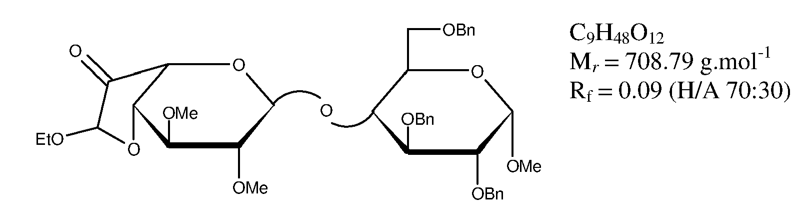

Through a solution of compound **Vllb** (145 mg, 0.21 mmol, 1.0 eq.) in anhydrous dichloromethane (10 mL), under an argon atmosphere and cooled to -78°C, was bubbled ozone (0.2 L/min, 110 V). When the solution had turned dark blue, oxygen was bubbled through in order to remove the excess ozone. When the solution became colorless dimethylsulfide (4 drops) was added and the solution was brought to room temperature. After 30 min stirring the reaction mixture was concentrated. Column chromatography (gradient hexane/ethyl acetate 90:10 - 60:40) afforded compound **Vlllb** as a white solid (100 mg, <67%) in a mixture of diastereomers (67:33).

**¹H NMR** (CDCl₃, 400 MHz): δ 1.21-1.28 (m, 3H), 2.93-3.07 (m, 1 H), 3.31-4.26 (m, 20H), 4.52-5.02 (m, 9H), 7.20-7.45 (m, 15H).

**ESI-MS** (pos. mode): m/z= 731 [M+Na]⁺.

### Preparation of Methyl-2,3,6-tri-O-benzyl-4-O(methyl 2',3'-di-O-methyl-α-L-idopyranosiduronate)-α-D-glucopyranoside (IXb)

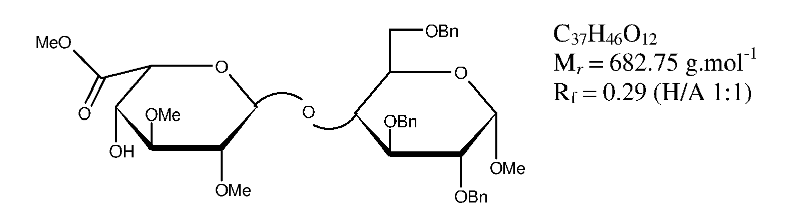

To a solution of compound **Vlllb** (62 mg, 87 µmol, 1.0 eq.) in dichloromethane (5 mL), under an argon atmosphere and cooled to 0°C, were added *m*-CPBA (77%, 58 mg, 259 µmol, 3.0 eq.) and NaHCO₃ (11 mg, 130 µmol, 1.5 eq.). After 5 hours stirring the solvent was removed under vacuum. The reaction mixture was then dissolved in EtOAc (50 mL) and washed successively with a 5% NaHCO₃ aqueous solution (1 x 10 mL), a 5 % citric acid aqueous solution (1 x 10 mL) and brine (1 x 10 mL). The organic layer was dried (MgSO₄), filtered and concentrated. The crude mixture was dissolved in anhydrous methanol (10 mL) and sodium methoxide was added to reach pH = 10. After 30 minutes stirring at room temperature the reaction mixture was neutralized with Dowex^{®}, filtered through a pad of Celite^{®}, and concentrated. The residue obtained was dissolved in DMF (10 mL) then triethylamine (13 µL, 93 µmol, 1.1 eq.) and methyl iodide (27 µL, 434 µmol, 5.0 eq.) were added. After 2h30 stirring the reaction mixture was concentrated, dissolved in EtOAc (40 mL) and washed with a 5% citric acid aqueous solution (2 x 10 mL), a 5% NaHCO₃ aqueous solution (2 x 10 mL), and brine (1 x 10 mL). The organic layer was dried (MgSO₄), filtered and concentrated. Column chromatography (gradient hexane/ethyl acetate 60:40-50:50) afforded compound **IXb** as a colourless oil (12 mg, 18 µmol, 20% over two steps).

**¹H NMR** (CDCI₃, 400 MHz): δ 3.23 (s, 3H), 3.20-3.25 (m, 1 H), 3.36 (s, 3H), 3.43 (s, 3H), 3.46 (s, 3H), 3.33-3.58 (m, 3H), 3.60-3.69 (m, 2H), 3.72-3.95 (m, 4H), 4.53-4.60 (m, 4H), 4.68-4.97 (m, 4H), 5.14 (s, 1 H), 7.24-7.37 (15H).

**ESI-MS** (pos. mode): m/z= 705 [M+Na]⁺.

## Claims

1. Process for preparing a compound of formula (VII) : 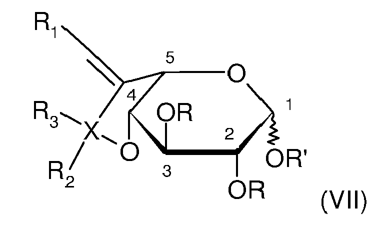 comprising conducting a decarboxylation/intramolecular cyclisation tandem reaction, which allows the inversion of configuration of carbon C5, from a compound of formula (VI) : 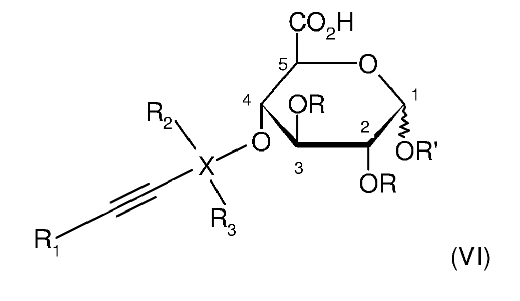 wherein :
- R, each being independently of each other identical or different, chosen from the group consisting in : alkyl, aryl and -arylalkyl;
- R' is chosen from the group consisting in : alkyl, aryl, -arylalkyl and oligosaccharide;
- R₁ represents H or alkyl;
- R₂ represents H or -O-alkyl;
- R₃ represents H or -O-alkyl;
- X represents C or Si; provided that when X represents Si, then R₃ is alkyl or when X represents C, then C is an asymmetric carbon which possesses either the (R) or the (S) configuration;
- 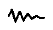 represents either α or β.

2. Process according to claim 1, where in formulae (VI) and (VII) :
- R₁ represents H; and/or
- R₂ represents -O-ethyl; and/or
- R₃ represents H; and/or
- X is C; and/or
- R' represents methyl or an oligosaccharide as defined in claim 1; and/or
- R represents methyl.

3. Process according to claim 1 or 2, wherein compounds of formulae (VI) and (VII) are of formulae: 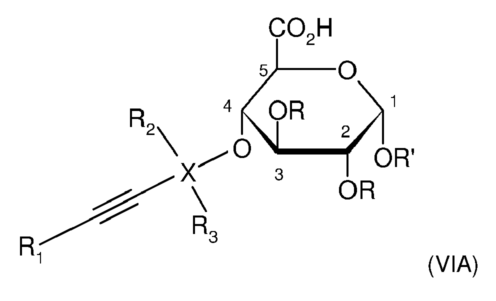 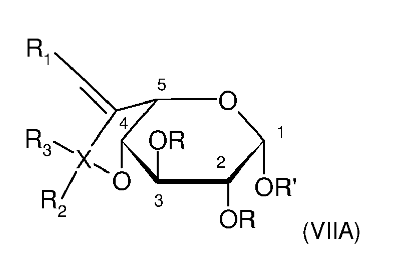 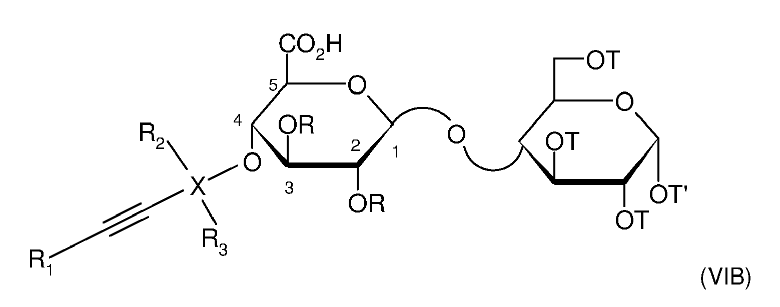 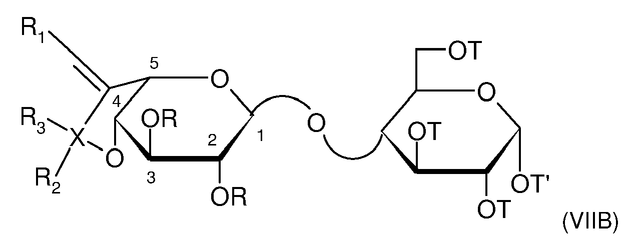 wherein 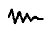 , R, X, R₁, R₂ and R₃ are as defined in claim 1, and T and T' are independently of each other identical or different, chosen from the group consisting in : alkyl, aryl and -arylalkyl.

4. Process for preparing a compound of formula (IX) : 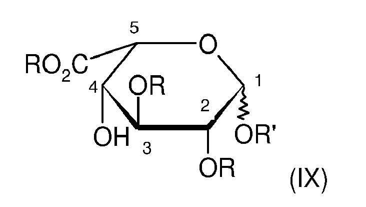 comprising reacting a compound of formula (IV): 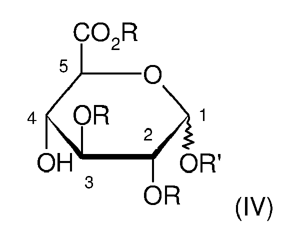 in which 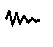 , R and R' are as defined in anyone of claims 1 to 3;
wherein said process comprises the process of preparation of a compound of formula (VII) from a compound of formula (VI) by decarboxylation/intramolecular cyclisation tandem reaction, according to anyone of claims 1 to 3.

5. Process according to anyone of claims 1 to 4, wherein the decarboxylation/intramolecular cyclisation tandem reaction is carried out under UV irradiations.

6. Process according to anyone of the preceding claims, wherein the decarboxylation is a radical decarboxylation chosen from the Barton decarboxylation, Kolbe decarboxylation or the decarboxylation via a *N-*(acyloxy)phthalimide.

7. Process according to claim 6, wherein the decarboxylation is a Barton decarboxylation.

8. Process according to anyone of claims 4 to 7, which comprises preparing the compound of formula (VI) as defined in claims 1, 2 or 3, comprising:
a. the transacetalisation of the compound of formula (IV) as defined in claim 4, for preparing a compound of formula (V) : 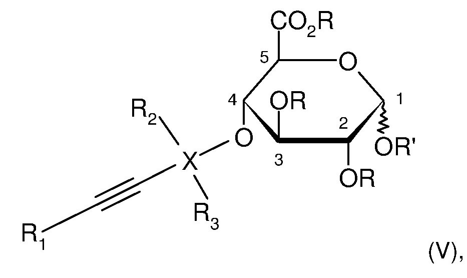 and
b. the saponification of the compound of formula (V) resulting from the step a) for preparing the compound of formula (VI): 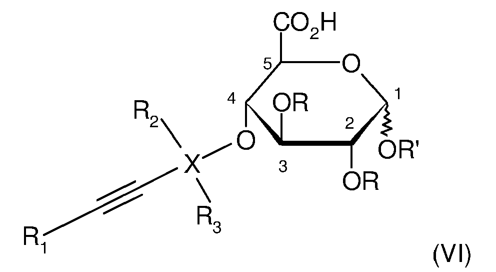 wherein 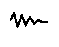 , R, R', X, R₁, R₂ and R₃ are as defined in anyone of claims 1 to 3.

9. Process according to anyone of claims 4 to 8, wherein the compound of formula (IV) : 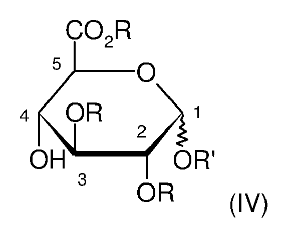 is prepared by an oxidation/esterification sequential reactions of a compound of formula (III) : 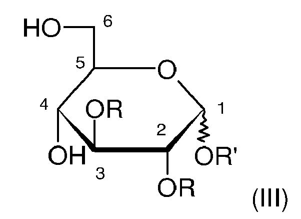 wherein 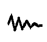 and R and R' are as defined in anyone of claims 1 to 3.

10. Process according to claim 9, wherein the compound of formula (III) : 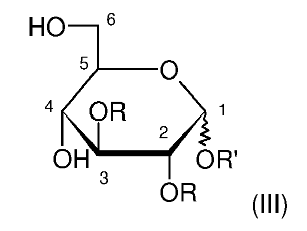 wherein R is as defined in anyone of claims 1 to 3 and R' is chosen among the group consisting in : alkyl, aryl and -arylalkyl;
is prepared by cleavage of the 4,6-*O*-benzylidene protecting group of a compound of formula (II) : 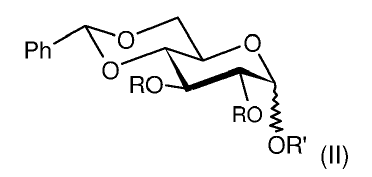 wherein 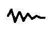 and R are as defined in anyone of claims 1 to 3, and R' is chosen among the group consisting in : alkyl, aryl and -arylalkyl.

11. Process according to claim 10, wherein the compound of formula (II) : 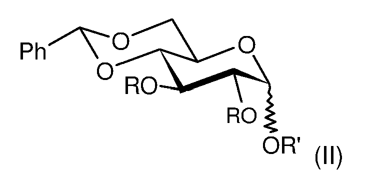 is prepared by O-alkylation of a compound of formula (1): 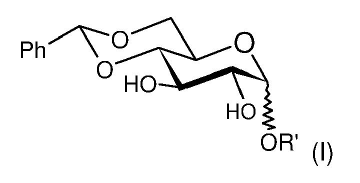 wherein 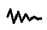 and R are as defined in anyone of claims 1 to 3, and R' is chosen among the group consisting in : alkyl, aryl and -arylalkyl.

12. Process according to claim 11, wherein the compound of formula (1) : 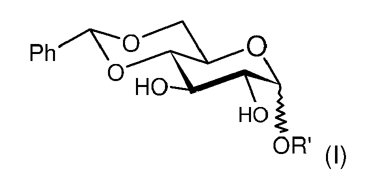 is prepared by 4,6-*O*-benzylidene protection of a compound of formula (A) 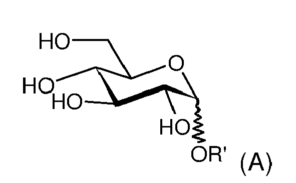 wherein 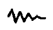 and R are as defined in anyone of claims 1 to 3, and R' is chosen among the group consisting in : alkyl, aryl and -arylalkyl.

13. Process according to anyone of the precedent claims, which further comprises:
a. the ozonolysis of the compound of formula (VII) : 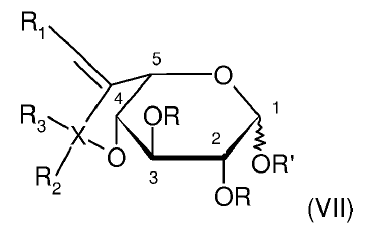 for preparing a compound of formula (VIII): 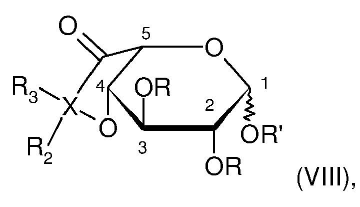 and
b. the oxidation/esterification sequential reactions of the compound of formula (VIII) resulting from step a), for preparing the compound of formula (IX) : 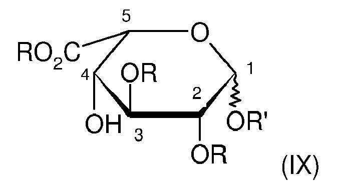 wherein 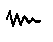 , R, R', X, R₁, R₂ and R₃ are as defined in anyone of claims 1 to 3.

14. Process for the inversion of configuration of carbon C5 from a compound of formula (IV) : 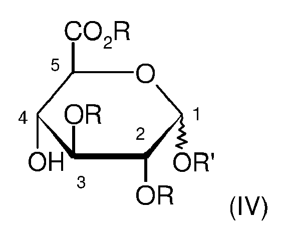 into a compound of formula (IX) : 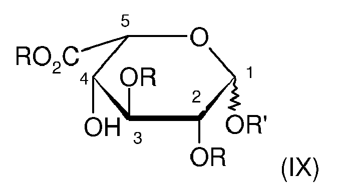 wherein 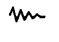 , R and R' are defined in anyone of claims 1 to 3; which comprises the process according to anyone of claims 1 to 13.

15. Compound of formula (V) : 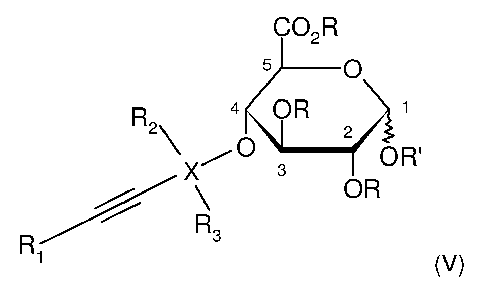 wherein 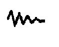 , R, R', X, R₁, R₂ and R₃ are as defined in anyone of claims 1 to 3.

16. Compound of formula (V) according to claim 15, of formula (Va) or (Vb) :

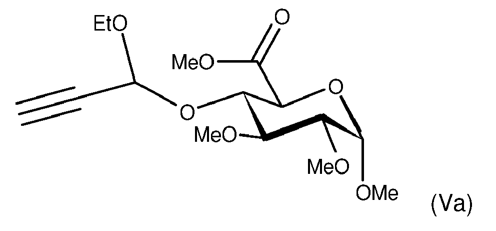

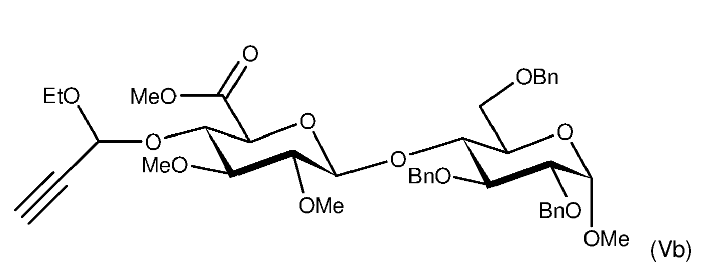

17. Compound of formula (VI) : 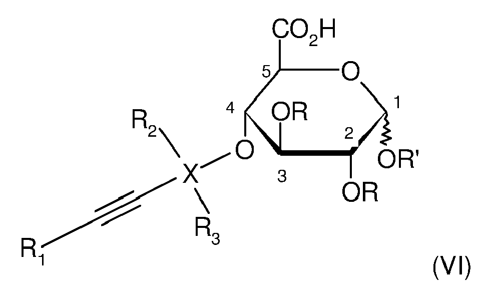 wherein 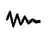 , R, R', X, R₁, R₂ and R₃ are as defined in anyone of claims 1 to 3.

18. Compound of formula (VI) according to claim 17, of formula (Vla) or (Vlb):

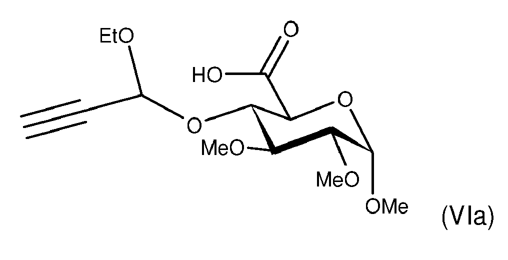

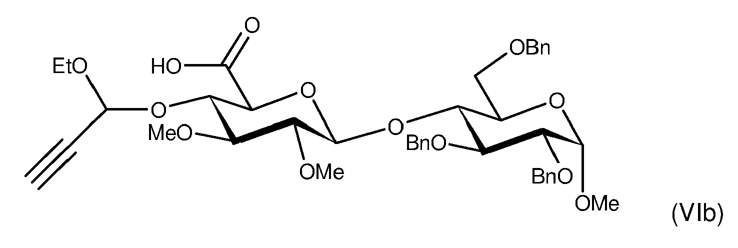

19. Compound of formula (VII) : 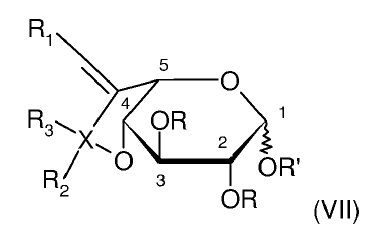 wherein 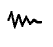 , R, R', X, R₁, R₂ and R₃ are as defined in anyone of claims 1 to 3.

20. Compound of formula (VII) according to claim 19, of formula (VIIa) or (Vllb):

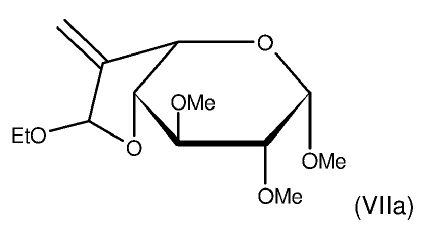

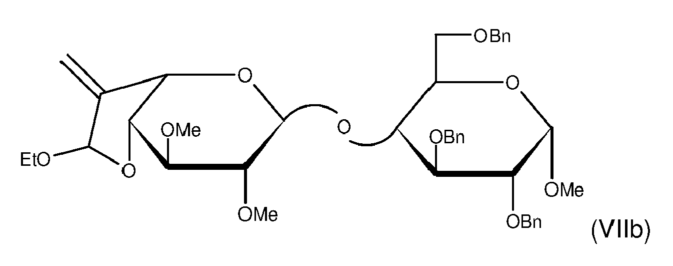

21. Compound of formula (VIII) : 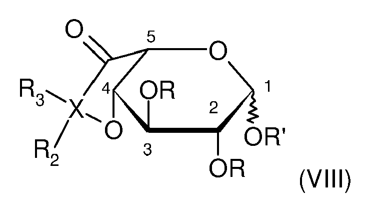 wherein 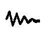 , R, R', X, R₂ and R₃ are as defined in anyone of claims 1 to 3.

22. Compound of formula (VIII) according to claim 21, of formula (VIIIa) or (Vlllb) :

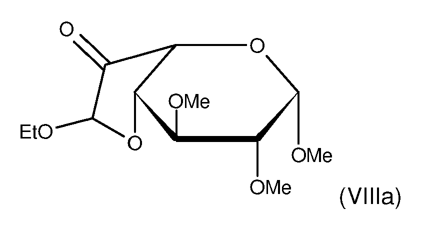

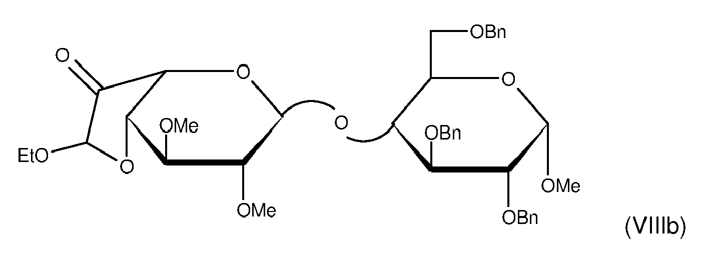

23. Compound of formula (IX) : 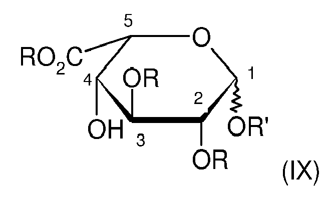 wherein 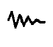 is as defined in anyone of claims 1 to 3, R and R' are as defined in anyone of claims 1 to 3;
except the compound wherein R represents methyl and R' represents the monosaccharide. of formula : 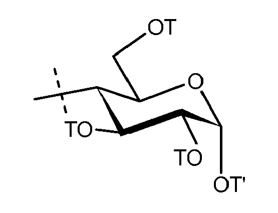 wherein T is benzyl and T' is methyl.

24. Compound of formula (IX) according to claim 23, of formula (IXa) :

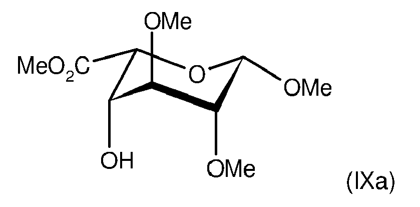

25. Process of preparation of Idraparinux comprising the following steps :
1) preparing a compound of formula (IXB) wherein R is methyl, T is benzyl and T' is methyl, by the process as defined in anyone of claims 1 to 13;
2) epimerisation of the disaccharide (IXB) so as to form disaccharide D of formula : 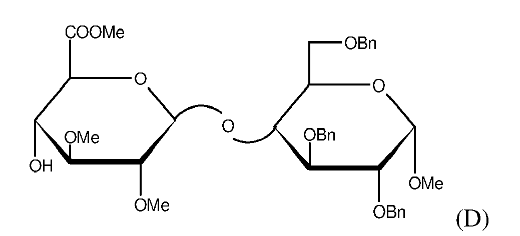
3) protection of the 4'-OH of D with a levulinoyl ester;
4) acetolysis of the disaccharide resulting from step 3), followed by preparation of the corresponding imidate;
5) coupling the disaccharide imidate resulting from step 4) with (IXB) obtainable by the process of the invention, wherein R is methyl, T is benzyl and T' is methyl, to obtain a tetrasaccharide;
6) coupling the fully protected tetrasaccharide with a monosaccharide glycosyl imidate;
7) deprotection of the protecting groups by the successive saponification and hydrogenolysis;
8) sulfation of the hydroxyl groups.

26. Use of a compound according to anyone of claims 15, 17, 19, 21 and 23, where R1, R2, R3 and X are defined as in anyone of claims 1 to 3, R is methyl and R' is the monosaccharide of formula : 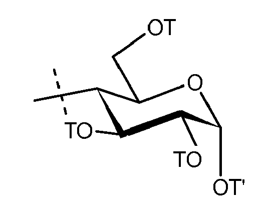 wherein T is benzyl and T' is methyl, as an intermediate for the preparation of Idraparinux.
